# EUROPEAN PATENT APPLICATION

(11) **EP 4 458 967 A1**
(43) Date of publication of application: **06.11.2024**
(21) Application number: 23171052.6
(22) Date of filing: 02.05.2023
(51) Int. Cl.: C12N 15/11, C12N 15/86, A61K 31/7105

(54) **PHARMACEUTICAL COMPOSITION COMPRISING MULTIPLE SUPPRESSOR TRANSFER RNAS**

(71) Applicant: Universität Hamburg, 20148 Hamburg (DE)
(72) Inventor: IGNATOVA, Zoya, D-20146 Hamburg (DE); BHARTI, Nikhil, D-20146 Hamburg (DE); DAVYT, Marcos, D-20146 Hamburg (DE)
(74) Representative: Stüven, Ralf

(57) **Abstract**

The invention relates, in one aspect, to pharmaceutical composition comprising at least five different suppressor transfer RNAs, wherein
a) at least one of the suppressor transfer RNAs is able to base-pair to a UGA stop codon, at least one of the suppressor transfer RNAs is able to base-pair to a UAA stop codon, and at least one of the suppressor transfer RNAs is able to base-pair to a UAG stop codon; and
b) the suppressor transfer RNAs are not all present in the composition in the same amount;
and a pharmaceutically acceptable carrier.

## Description

The invention relates to a pharmaceutical composition comprising multiple suppressor transfer RNAs and to a synthetic DNA construct comprising nucleic acids encoding multiple suppressor transfer RNAs. Further, the invention relates to a pharmaceutical composition comprising one or more synthetic DNA constructs encoding suppressor tRNA, that can be used, for example, for the delivery of multiple transfer RNA into cells, for example human cells.

Transfer ribonucleic acids (tRNAs) are an essential part of the protein synthesis machinery of living cells as necessary components for translating the nucleotide sequence of a messenger RNA (mRNA) into the amino acid sequence of a protein. Naturally occurring tRNAs comprise an amino acid binding stem being able to covalently bind an amino acid and an anticodon loop containing a base triplet called "anticodon", which can bind non-covalently to a corresponding base triplet called "codon" on an mRNA. A protein is synthesized by assembling the amino acids carried by tRNAs using the codon sequence on the mRNA as a template with the aid of a multi component system comprising, inter alia, the ribosome and several auxiliary enzymes.

Transfer RNAs have recently seen increasing interest in their use as drugs for therapeutic purposes, e.g. as part of a gene therapy for treating conditions associated with nonsense mutations, i.e. mutations changing a sense codon encoding one of the twenty amino acids specified by the genetic code to a chain-terminating codon ("premature termination codon", PTC) in a gene sequence (Ai-Ming Yu, Young Hee Choi and Mei-Juan Tu, RNA Drugs and RNA Targets for Small Molecules: Principles, Progress, and Challenges, Pharmacological Reviews, 2020, 72 (4) 862-898; DOI: 10.1124/pr. 120.019554; Porter, JJ, Heil, CS, Lueck, JD, Therapeutic promise of engineered nonsense suppressor tRNAs, WIREs RNA. 2021; 12:e1641, DOI: 10.1002/wrna.1641; Albers S, Beckert B, Matthies MC, Mandava CS, Schuster R, Seuring C, Riedner M, Sanyal S, Torda AE, Wilson DN, Ignatova Z., Repurposing tRNAs for nonsense suppression. Nat Commun. 2021 Jun 22;12(1):3850. doi: 10.1038/s41467-021-24076-x. PMID: 34158503). Lueck et al. 2016 (Lueck, J.D., Infield, DT, Mackey, AL, Pope, RM, McCray, PB, Ahern, CA. Engineered tRNA suppression of a CFTR nonsense mutation, bioRxiv 088690; doi: 10.1101/088690; Lueck JD, Yoon JS, Perales-Puchalt A, Mackey AL, Infield DT, Behlke MA, Pope MR, Weiner DB, Skach WR, McCray PB Jr, Ahern CA. Engineered transfer RNAs for suppression of premature termination codons. Nat Commun. 2019 Feb 18;10(1):822. doi: 10.1038/s41467-019-08329-4), for example, describe a codon-edited tRNA enabling the conversion of an in-frame stop codon in the CFTR gene to the naturally occurring amino acid in order to restore the full-length wild type protein.

Compared to mRNA, tRNA molecules offer significantly higher stability and are on average 10-fold shorter, alleviating the problem of introduction into the target tissue. This has led to attempts to use tRNA in gene therapy in order to prevent the formation of a truncated protein from an mRNA with a premature stop codon and to introduce the correct amino acid instead (see, e.g., Koukuntla, R., 2009, Suppressor tRNA mediated gene therapy, Graduate Theses and Dissertations, 10920, Iowa State University, http://lib.dr.iastate.edu/etd/10920; US 2003/0224479 A1; US 6964859). WO 2017/121863 A1 and WO 2020/208169 A1 describe synthetic tRNA with an extended anticodon loop that can, for example, be used as a suppressor tRNA for genetic diseases associated with a frameshift mutation. WO 2021/113218 A1 discloses engineered tRNA molecules and vectors encoding engineered suppressor tRNA molecules that recognize and readthrough of disease-causing premature stop codons. The use of tRNA in the context of gene therapy, e.g., of diseases associated with the existence of premature termination codons (PTCs), for example, is also described, inter alia, in WO 2020/069194 A1, WO 2021/211762 A2, WO 2021/087401 A1, WO 2021/113218 A1, and US 2020/291401 A1.

Although the symptoms of a disease like, for example, cystic fibrosis, caused by or associated with a nonsense mutation (PTC) in a gene coding for a protein, leading to the lack of the protein or the protein being non-functional, are often quite similar, the genetic signature of such a disease can be highly diverse. Each patient, although showing the same disease phenotype, may have a unique genetic mutation pattern underlying the disease, with one or more different PTCs. It would, however, be desirable to have a single medication for the treatment of at least several patients or a larger patient population having the same or similar disease phenotype, but having different PTC mutations or PTC mutation patterns, or similar underlying genetic mutations but in another gene associated with another disease. In WO 2022/235861 A1, it is suggested to use a single expression vector or a pharmaceutical composition comprising a first, second, and/or third suppressor tRNA.

It is an object of the invention to improve the means and possibilities for gene therapy of a disease caused or mediated by nonsense mutations in a gene using transfer RNA. In particular, it is an object of the invention to enable the treatment of as many patients as possible having the same disease phenotype but having a different underlying genotypic nonsense mutation pattern.

For solving the problem, the invention provides, in a first aspect, a pharmaceutical composition comprising at least five different suppressor transfer RNAs, wherein
a) at least one of the suppressor transfer RNAs is able to base-pair to a UGA stop codon, at least one of the suppressor transfer RNAs is able to base-pair to a UAA stop codon, and at least one of the suppressor transfer RNAs is able to base-pair to a UAG stop codon; and
b) the suppressor transfer RNAs are not all present in the composition in the same amount: and a pharmaceutically acceptable carrier.

The invention provides, in the above first aspect, a pharmaceutical composition that can be used to treat a variety of patients having a nonsense mutation (PTC) related disease or being diagnosed with at least one nonsense mutation associated with a disease. The composition contains at least five suppressor transfer RNAs, three of which are configured for the suppression of the three possible PTC (UGA, UAG, UAA) in a mutated gene of interest. The suppressor transfer RNA present in the pharmaceutical composition of the invention differ from each other, be it in the cognate amino acid and/or the targeted PTC, i.e. each of the suppressor transfer RNAs of the pharmaceutical composition has a unique combination of cognate amino acid and target PTC that distinguishes it from the other suppressor transfer RNAs in the composition. At least one of the suppressor transfer RNAs is present in the pharmaceutical composition in an effective amount. The pharmaceutically acceptable carrier can be any carrier suitable for pharmaceutical purposes. An example of a simple carrier is a buffer solution or a physiologic salt solution.

The pharmaceutical composition of the invention can be tailored to the disease and the specific PTC pattern of a patient in that the type and proportion of the suppressor tRNAs can be specifically adapted. With the pharmaceutical composition of the invention, it is, for example, not necessary to go through approval procedures for a large number of different compositions. It would be sufficient to seek for approval of a composition containing a maximal therapeutic amount of each of the suppressor transfer RNAs to be administered. It might even be possible to conduct clinical trials and seek approval for a single composition containing all suppressor transfer RNAs in a maximum therapeutic amount, which could be administered in order to treat all or at least a variety of diseases and patients. It would also be easier to produce and design customized pharmaceutical compositions, i.e. pharmaceutical compositions customized in relation to the disease and/or the patient's PTC pattern. Customization may involve the selection of the suppressor transfer RNAs in terms of targeted PTC and cognate amino acid, and/or a suitable proportioning of the suppressor transfer RNAs, depending on the disease and/or the PTC pattern of the patient.

The inventors have surprisingly found that it would, for example, be sufficient to have only three different pharmaceutical suppressor tRNA compositions (suppressor tRNA "cocktails") in order to treat all or at least almost all known nonsense mutations, and thus a broad variety of PTC related diseases. The suppressor tRNA compositions differ in their composition in regards to the suppressor tRNAs, so that each of the suppressor tRNAs is only present in one of the pharmaceutical suppressor tRNA compositions. Each pharmaceutical suppressor tRNA composition preferably includes a maximum of six suppressor tRNAs, selected from a total of 19 suppressor transfer RNAs (see below).

The terms "DNA construct" or "synthetic DNA construct" relate to an artificially-designed segment of DNA that can, for example, be used to incorporate genetic material into a target tissue or cell. The terms "vector", "synthetic vector" or "synthetic gene delivery vehicle (GDV)" refer to any means for delivering a nucleic acid, for example a coding nucleic acid together with regulatory elements like promoter sequences and termination signals, into a living cell. Many viral and non-viral vectors are known. Examples are plasmids, viruses, cationic liposomes, nanoparticles or polymers etc.

For the term "nucleic acid encoding a transfer RNA" the terms "transfer RNA gene" or "tRNA gene" may also be used synonymously.

The term "encoding" in relation to a nucleotide sequence of a tRNA gene means that the nucleotide sequence is transcribed into a tRNA or part of a tRNA. A direct reference to a tRNA encoded by the DNA construct of the invention, e.g., a reference to a structural part of the mature tRNA, for example, the D arm, anticodon arm, T arm or acceptor stem, is to be understood as referring to the regions of the tRNA gene in the nucleic acid encoding those structural parts, unless clearly stated otherwise or clearly recognizable from the context. A wording according to which an encoded tRNA comprises, for example, a T arm of a specific sequence, the sequence presented as a DNA (with the nucleotides A, C, G, and T), is thus to be understood as referring to the sequence within the tRNA gene coding for the corresponding structure, wherein the corresponding structure appears in the mature tRNA transcribed from the tRNA gene, however, with T replaced by U, and, as the case may be, with modified nucleotides.

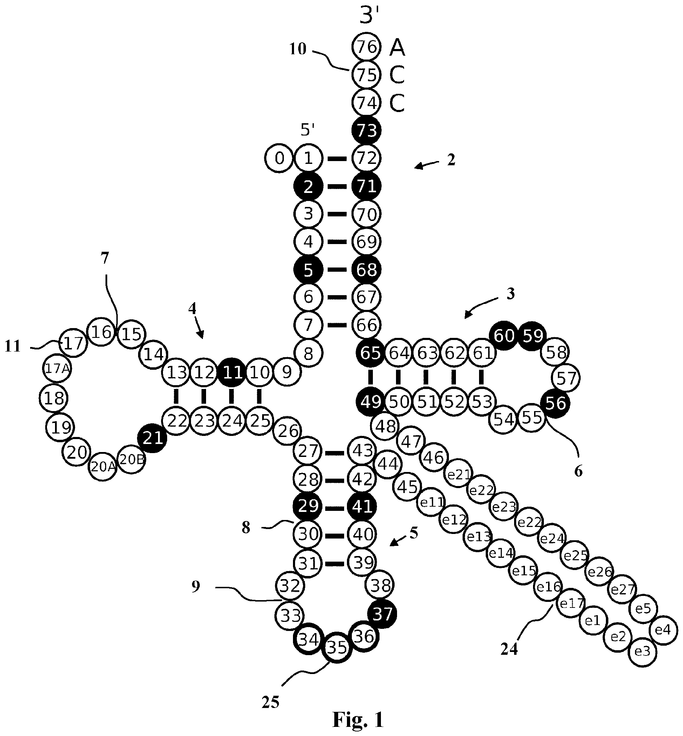

The terms "transfer ribonucleic acid", "transfer RNA" or "tRNA" refer to RNA molecules with a length of typically 73 to 90 nucleotides, which mediate the translation of a nucleotide sequence in a messenger RNA into the amino acid sequence of a protein. tRNAs are able to covalently bind a specific amino acid at their 3' CCA tail at the end of the acceptor stem, and to base-pair via a usually three-nucleotide anticodon in the anticodon loop of the anticodon arm with a usually three-nucleotide sequence (codon) in the messenger RNA. Some anticodons can pair with more than one codon due to a phenomenon known as wobble base pairing. The secondary "cloverleaf' structure of tRNA comprises the acceptor stem binding the amino acid and three arms ("D arm", "T arm" and "anticodon arm") ending in loops (D loop, T loop (TψC loop), anticodon loop), i.e. sections with unpaired nucleotides. The terms "D stem", "T stem" (or "TψC stem") and "anticodon stem" (also "AC stem") relate to portions of the D arm, T arm and anticodon arm, respectively, with paired nucleotides. Aminoacyl tRNA synthetases charge (aminoacylate) tRNAs with a specific amino acid. Each tRNA contains a distinct anticodon triplet sequence that can base-pair to one or more codons encoding an amino acid. By convention, the nucleotides of tRNAs are often numbered 1 to 76, starting from the 5'-phosphate terminus, based on a "consensus" tRNA molecule consisting of 76 nucleotides, and regardless of the actual number of nucleotides in the tRNA, which are not always of a length of 76 nt due to variable portions, such as the D loop or the variable loop in the tRNA (see Fig. 1).

Following this convention, nucleotide positions 34-36 of naturally occurring tRNA refer to the three nucleotides of the anticodon, and positions 74-76 refer to the terminating CCA tail. Any "supernumerary" nucleotide can be numbered by adding alphabetic characters to the number of the previous nucleotide being part of the consensus tRNA and numbered according to the convention, for example 20a, 20b etc. for the D-arm, or by independently numbering the nucleotides and adding a leading letter, as in case of the variable loop such as e11, e12 etc. (see, for example, Sprinzl M, Horn C, Brown M, Ioudovitch A, Steinberg S. Compilation of tRNA sequences and sequences of tRNA genes. Nucleic Acids Res. 1998;26(1): 148-53). In the following, the tRNA-specific numbering will also be referred to as "tRNA numbering convention" or "transfer RNA numbering convention".

The term "tRNA body" refers to the portion of the tRNA outside the anticodon loop.

The term "intron" relates to a polynucleotide sequence in a nucleic acid that is part of the nucleic acid within a genome and within a first nucleic acid product transcribed from the genomic nucleic acid, but is not contained in the final nucleic acid product. In case of a protein gene, for example, an intron is a non-coding polynucleotide sequence separating coding polynucleotide sequences (exons), which is excised from the pre-mRNA transcribed from the protein gene in a process called "splicing". In case of a transfer RNA, for example, the term intron relates to a polynucleotide sequence that is excised (spliced) from a pre tRNA transcribed from the tRNA gene.

The term "intronic tRNA" relates to a tRNA, the precursor (pre-tRNA) of which contains an intron that is spliced from the pre-tRNA during processing of the pre-tRNA into the final (mature) tRNA. The term "non-intronic tRNA" relates to tRNA being generated from pre-tRNAs that do not contain an intron and do not undergo splicing. The terms "intronic tRNA" or "non-intronic tRNA" encompass the pre-tRNA and the mature tRNA.

The term "tDNA", if used herein, relates to a sequence of DNA encoding a tRNA, in particular a DNA sequence having the sequence of a tRNA wherein the uracil nucleotides (U) of the tRNA are replaced with thymine nucleotides (T).

The terms "engineered transfer ribonucleic acid" or "synthetic tRNA" refer to a tRNA modified by chemical or molecular biological methods or a non-naturally occurring tRNA. The terms "engineered" and "synthetic" are used synonymously here. An example is a tRNA that will be aminoacylated with an amino acid under natural conditions, but has an anticodon pairing to a stop codon instead of the anticodon for the corresponding amino acid.

The term "codon" refers to a sequence of nucleotide triplets, i.e. three DNA or RNA nucleotides, corresponding to a specific amino acid or stop signal during protein synthesis. A list of codons (on mRNA level) and the encoded amino acids are given in the following:

| Amino acid | Three Letter Code | One Letter Code | Codons |
|---|---|---|---|
| alanine | Ala | A | GCU, GCC, GCA, GCG |
| arginine | Arg | R | CGU, CGC, CGA, CGG, AGA, AGG |
| asparagine | Asn | N | AAU, AAC |
| aspartic acid | Asp | D | GAU, GAC |
| cysteine | Cys | C | UGU, UGC |
| glutamine | Gln | Q | CAA, CAG |
| glutamic acid | Glu | E | GAA, GAG |
| glycine | Gly | G | GGU, GGC, GGA, GGG |
| histidine | His | H | CAU, CAC |
| isoleucine | Ile | I | AUU, AUC, AUA |
| leucine | Leu | L | UUA, UUG, CUU, CUC, CUA, CUG |
| lysine | Lys | K | AAA, AAG |
| methionine | Met | M | AUG |
| phenylalanine | Phe | F | UUU, UUC |
| proline | Pro | P | CCU, CCC, CCA, CCG |
| serine | Ser | S | UCU, UCC, UCA, UCG, AGU, AGC |
| threonine | Thr | T | ACU, ACC, ACA, ACG |
| tryptophan | Trp | W | UGG |
| tyrosine | Tyr | Y | UAU, UAC |
| valine | Val | V | GUU, GUC, GUA, GUG |

### START: AUG

### STOP: UAA, UGA, UAG, abbreviated "X"

The term "sense codon" as used herein refers to a codon coding for an amino acid. The term "stop codon" or "nonsense codon" refers to a codon, i.e. a nucleotide triplet, of the genetic code not coding for one of the 20 amino acids normally found in proteins and signalling the termination of translation of a messenger RNA.

The term "5' leader sequence" as used herein refers to a sequence of nucleotides located "upstream" of the 5' end of a tRNA gene, for example approximately 100 nt upstream of the 5' end of the coding sequence for the mature tRNA, comprising a nucleotide sequence functioning as a binding site for the transcription factor TFIIIB. The term "5' leader sequence" refers in particular to the sequence of nucleotides comprising or consisting of positions -100 to -1 5' from the transcription start of a tRNA gene. "Mature tRNA" refers to a fully processed and functional tRNA. The terms "extragenic leader sequence", "5' untranslated region (5' UTR) or "extragenic pol III binding sequence" may also be used here.

The terms "A box" and "B box" refer to intragenic (gene internal) regions, i.e., sequence motifs of a tRNA gene comprising nucleotide sequences to which the transcription factor TFIIIC of the RNA polymerase III binds. The boxes may also be considered as part of a tRNA promoter (so-called type 2 promoter) or promoter elements. The nucleotide sequence of about 10-14 nucleotides in length (Mitra S., Das P., Samadder A., Das S., Betai R., Chakrabarti J., 2015, Eukaryotic tRNAs fingerprint invertebrates vis-à-vis vertebrates, Journal of Biomolecular Structure and Dynamics, doi: 10.1080/07391102.2014.990925) forming the A box are located in the region of the tRNA gene encoding part of the D arm, the nucleotide sequence of about 11 nucleotides in length (Mitra S., Das P., Samadder A., Das S., Betai R., Chakrabarti J., 2015, Eukaryotic tRNAs fingerprint invertebrates vis-à-vis vertebrates, Journal of Biomolecular Structure and Dynamics, doi: 10.1080/07391102.2014.990925) forming the B box are located in the region of the tRNA gene encoding part of the T loop. An 11nt consensus sequence for the B-Box is given by Mitra et al. 2015 (see above) as being RGTTCRANNCY, covering the nucleotides N52-N62 of the mature tRNA. Any numbering in relation to a tRNA gene or part thereof, e.g., the A and B boxes, refers to the nucleotide numbering within the mature tRNA, following the tRNA numbering convention (see below).

The term "frameshift mutation" refers to an out-of-frame insertion or deletion (collectively called "indels") of nucleotides with a number not evenly divisible by three. This perturbs the nucleotide sequence decoding which proceeds in steps of three nucleotide bases. The term "-1 frameshift mutation" relates to the deletion of a single nucleotide causing a shift in the reading frame by one nucleotide leading to the first nucleotide of the following codon being read as part of the codon from which the nucleotide has been deleted. Deletion of one nucleotide from the upstream codon along with several triplets (i.e. deletion of 4, 7, 10 etc. nucleotides) is also considered as -1 frameshifting. The term "+1 frameshift mutation" relates to the insertion of a single nucleotide into a triplet, or the deletion of two nucleotides. The result of either event is to shift the reading frame by one nucleotide, such that a nucleotide of an upstream codon is being read as part of a downstream codon. Insertion of one nucleotide along with several triplets (3*n*+1 nucleotides, n being an integer, i.e. insertion of 4, 7, 10 etc. nucleotides), or deletion of two nucleotides from the upstream codon along with several triplets (i.e. deletion of 5, 8, 11 etc nucleotides) is also considered as +1 frameshifting.

The term "anticodon" refers to a sequence of usually three nucleotides within a tRNA that base-pair (non-covalently bind) to the three bases (nucleotides) of the codon on the mRNA. In a natural tRNA a standard three-nucleotide anticodon is usually represented by the nucleotides at positions 34, 35 and 36. An anticodon may also contain nucleotides with modified bases. The terms "four nucleotide anticodon" or "four base anticodon" relate to an anticodon having four consecutive nucleotides (bases) which pair with four consecutive bases on an mRNA. The terms "quadruplet nucleotide anticodon" or "quadruplet anticodon" may also be used to denote a "four nucleotide anticodon". The terms "five nucleotide anticodon" or "five base anticodon" relate to an anticodon having five consecutive nucleotides (bases) which bind (base-pair) to five consecutive bases on an mRNA. The terms "quintuplet nucleotide anticodon" or "quintuplet anticodon" may also be used to denote a "five nucleotide anticodon". The term "stop anticodon" refers to an anticodon binding to one of the stop codons.

The term "anticodon arm" refers to a part of the tRNA comprising the anticodon. The anticodon arm is composed of a stem portion ("anticodon stem"), usually consisting of five base pairs (positions 27-31 and 39-43), and a loop portion ("anticodon loop"), i.e. a consecutive sequence of unpaired nucleotides bound to the anticodon stem. The anticodon arm takes the positions 27 to 43, the position numbering following transfer RNA numbering convention.

The term "anticodon loop" refers to the unpaired nucleotides of the anticodon arm containing the anticodon. Naturally occurring tRNAs usually have seven nucleotides in their anticodon loop, three of which pair to the codon in the mRNA.

The term "extended anticodon loop" refers to an anticodon loop with a higher number of nucleotides in the loop than in naturally occurring tRNAs. An extended anticodon loop may, for example, contain more than seven nucleotides, e.g. eight, nine, or ten nucleotides. In particular, the term relates to an anticodon loop comprising an anticodon composed of more than three consecutive nucleotides, e.g. four, five or six nucleotides, being able to base-pair with a corresponding number of consecutive nucleotides in an mRNA.

The term "anticodon stem" refers to the paired nucleotides of the anticodon arm that carry the anticodon loop.

The term "T arm" relates to a portion of a tRNA composed of a stem portion ("T stem") and a loop portion ("T loop") between the acceptor stem and the variable loop. The T arm takes the positions 49 to 65, the position numbering following transfer RNA numbering convention. The T stem is usually composed of five nucleotide pairs (taking positions 49-53 and 61-65).

The term "T stem" (or "TψC" stem) relates to the paired nucleotides of the T arm, which carry the T loop, i.e. the unpaired nucleotides of the T arm.

The term "D arm" relates to the tRNA portion composed of a stem ("D stem"), i.e. the paired nucleotides of the D arm, and a D loop, i.e. the unpaired nucleotides of the D arm, between the acceptor stem and the anticodon arm. The D arm takes the positions 10-25, the position numbering following transfer RNA numbering convention.

The term "variable loop" refers to a tRNA loop located between the anticodon arm and the T arm. The number of nucleotides composing the variable loop may largely vary from tRNA to tRNA. The variable loop can thus be rather short or even missing, or rather large, forming, for example, a helix. The term "variable arm" may synonymously be used for the term "variable loop". Use of the term "variable loop" does not exclude the existence of a "stem" portion within the variable loop, i.e. a stretch of consecutive nucleotides of the variable loop forming base pairs with a complementary stretch of other consecutive nucleotides of the variable loop. The variable loop takes the positions 44 to 48, the position numbering following transfer RNA numbering convention. It should be noted that the variable loop may have a considerable number of additional nucleotides that are separately numbered (see Fig. 1).

The term "acceptor stem" relates to the site of attachment of amino acids to the tRNA. An acceptor stem is often formed by 7 base pairs.

The terms "codon base triplet" or "anticodon base triplet", when used herein, refer to sequences of three consecutive nucleotides which form a codon or anticodon. Synonymously, the terms "three nucleotide codon" (also "three base codon") or "three nucleotide anticodon" (also "three base anticodon"), or abbreviations thereof, e.g. "3nt codon" or "3nt anticodon", may be used.

The term "base pair" refers to a pair of bases, or the formation of such a pair of bases, joined by hydrogen bonds. The term "Watson-Crick base pair" may also be used for such a pair of bases. One of the bases of the base pair is usually a purine, and the other base is usually a pyrimidine. In RNA, for example, the bases adenine and uracil can form a base pair and the bases guanine and cytosine can form a base pair. In DNA thymine usually forms a base pair with adenine instead of uracil. However, the formation of other base pairs ("wobble base pairs") is also possible and encompassed by the term, e.g. base pairs of guanine-uracil (G-U), hypoxanthine-uracil (I-U), hypoxanthine-adenine (I-A), and hypoxanthine-cytosine (I-C). The term "being able to base-pair" refers to the ability of nucleotides or a sequence of nucleotides to hybridize to a complementary nucleotide or sequence of nucleotides, i.e. to form hydrogen-bond-stabilised structures with a corresponding (complementary) nucleotide or nucleotide sequence. Base pairing can occur intramolecular, e.g., within a single-stranded nucleic acid (e.g., a tRNA), or intermolecular, i.e., between different nucleic acid molecules. An expression, according to which a "suppressor transfer RNA is able to base-pair to a stop codon" means that the suppressor transfer RNA is able to bind, via its anticodon, to the stop codon.

The term "base", as used herein, for example in terms like "the bases A, C, G or U" or "the bases A, C, G or T", encompasses or is synonymously used to the term "nucleotide", unless the context clearly indicates otherwise.

Abbreviations used her for bases or nucleotides are according to the ILTPAC nucleotide code and standard ST.26 (version 1.5), as follows:

| IUPAC nucleotide code | Base |
|---|---|
| A | Adenine |
| C | Cytosine |
| G | Guanine |
| T (or U) | Thymine (or Uracil in RNA) |
| R | A or G |
| Y | C or T/U |
| S | G or C |
| W | A or T/U |
| K | G or T/U |
| M | A or C |
| B | C or G or T/U |
| D | A or G or T/U |
| H | A or C or T/U |
| V | A or C or G |
| N | any base |
| . or - | gap |

The term "PTC" refers to a premature termination codon. This is a stop codon introduced into a coding nucleic acid sequence by a nonsense mutation, i.e. a mutation in which a sense codon coding for one of the twenty proteinogenic amino acids specified by the standard genetic code is changed to a chain-terminating codon. The term thus refers to a premature stop signal in the translation of the genetic code contained in mRNA. The terms "premature stop codon" ("PSC") or "nonsense mutation" may be used synonymously for a premature termination codon, PTC. The term is not restricted to a PTC generated by nonsense mutation of a codon, i.e. mutation of nucleotides within a codon, but includes PTC generated by frameshift mutation, i.e. by the deletion or insertion of one or more nucleotides.

The terms "frameshift suppression" or "frameshift rescue" refer to mechanisms masking the effects of a frameshift mutation and at least partly restoring the wild-type phenotype.

The terms "nonsense suppression", "nonsense mutation suppression", "PTC suppression" or "PTC rescue" refer to mechanisms masking the effects of a nonsense mutation and at least partly restoring mRNA translation and the wild-type phenotype.

The term "tRNA sequestration" relates to the irreversible binding of a tRNA to a tRNA synthetase such that the tRNA binds to the tRNA synthetase, but is not or essentially not released from the tRNA synthetase.

The term "suppressor tRNA" (also abbreviated as "sup-tRNA) relates to a tRNA altering the reading of a messenger RNA in a given translation system such that, for example, a frameshift or nonsense mutation is "suppressed" and the effects of the frameshift or nonsense mutation are masked and the wild-type phenotype at least partly restored. An example of a suppressor tRNA is a tRNA carrying an amino acid and being able to base-pair to a PTC in an mRNA or, in case of a tRNA with an extended anticodon loop and a four-, five- or six-base anticodon, for example, to a section on an mRNA having two consecutive codons, one or both of which being mutated, or having a first and consecutive second codon, of which one is intact and one has an insertion or deletion. The translation system can thus correct the reading frame in the case of frameshift mutation or read through a PTC in the case of non-sense mutation.

The term "near-cognate suppressor tRNA" relates to a suppressor tRNA pairing, with its anticodon, to the same PTC but having another cognate amino acid. The term "having a cognate amino acid" in relation to a suppressor tRNA is not be construed as a restriction according to which a suppressor tRNA actually bears (is charged with) its cognate amino acid, but includes a suppressor tRNA that is structurally configured in such a manner that it is recognized, under suitable conditions, e.g. within a living cell, by an aminoacyl-tRNA synthetase (aaRSs) charging the tRNA with its cognate amino acid.

The term "targeted PTC" or a wording according to which a suppressor tRNA binds to or targets a PTC relates to the ability of the suppressor tRNA to bind, via its anticodon, to a specific PTC, i.e., a PTC having a sequence complementary to the anticodon, or at least allowing the anticodon of the suppressor tRNA to form sufficient Watson-Crick base pairs for binding to the PTC. A "targeted PTC" is a PTC to be rescued with the aid of the suppressor tRNA, i.e., a PTC to be read through. A "suppressor tRNA targeting a PTC" is a suppressor tRNA promoting PTC readthrough during translation.

The terms "PTC pattern", "PTC signature", "nonsense mutation pattern" or "nonsense mutation signature" relate to the distribution patterns of PTC, in particular the locations or combination of locations of PTC in a given gene or disease.

The terms "disease associated with a nonsense mutation", "disease caused or mediated by a PTC" or "PTC related disease" relates to diseases or conditions that are based on at least one nonsense mutation or a frameshift mutation leading to a PTC within a gene. Examples of such diseases are cystic fibrosis, Duchenne muscular dystrophy, hemophilia A, Spinal Muscular Atrophy, Hurler syndrome, and β-thalassemia.

The term "decoding activity" in relation to a tRNA refers to the property or capacity of a transfer RNA to be used by the translation machinery of a cell as an amino acid donor for the production of a protein. A tRNA has a "decoding activity" if, under physiologic conditions, it is charged with a cognate amino acid and the charged tRNA (aminoacyl-tRNA) is subsequently incorporated into a protein. The decoding activity of a first tRNA for a given amino acid can, for example, be compared with the decoding activity of a second tRNA for the same amino acid by comparing the proportion at which the amino acid of the first or second tRNA is incorporated in the protein. The terms "rescue activity", "suppression activity", "suppression efficiency" or "readthrough activity" refers to the decoding activity or efficiency of a suppressor tRNA, e.g. a frameshift suppressor or nonsense suppressor tRNA, in particular a tRNA designed to decode a premature stop codon in an mRNA into an amino acid, such that translation of the mRNA into the corresponding protein is not prematurely interrupted.

A term according to which a tRNA encoded by the DNA construct of the invention "still serves its function in the translational machinery of a living cell" relates to a tRNA transcribed from the tRNA gene having a decoding activity. In case of a suppressor tRNA, the term relates to a tRNA having a rescue activity greater than the spontaneous (stochastic) rescue activity in a given cellular environment.

The term "aminoacylation" refers to the enzymatic reaction in which a tRNA is charged with an amino acid. An aminoacyl tRNA synthetase (aaRS) catalyses the esterification of a specific cognate amino acid or its precursor to a compatible cognate tRNA to form an aminoacyl-tRNA. The term "aminoacyl-tRNA" thus refers to a tRNA with an amino acid attached to it. Each aminoacyl-tRNA synthetase is highly specific for a given amino acid, and, although more than one tRNA may be present for the same amino acid, there is only one aminoacyl-tRNA synthetase for each of the 20 proteinogenic amino acids. The terms "charge" or "load" may also be used synonymously for "aminoacylate".

The term "expression" refers to the conversion of a genetic information into a functional product, for example the formation of a protein or a nucleic acid, e.g. functional RNA (e.g., transfer RNA), on the basis of the genetic information. The term not only encompasses the biosynthesis of a protein, e.g., an enzyme, based on genetic information including previous processes such as transcription or splicing, i.e., the formation of mRNA based on a DNA template, but also the synthesis of a functional RNA molecule, for example a tRNA. In relation to the production of a mature tRNA in a cell from a tRNA gene, in particular in terms of increased or decreased production, the term "transcription" may be used synonymously to the term "expression", thus not only encompassing the synthesis of the first RNA product (i.e., the pre-tRNA) but also the processing of the first RNA product to the final RNA product (i.e., the mature tRNA). The terms "expression strength" or "expression level" relate to the amount of product synthesized from a DNA template. The term "high expression" relates to a high expression level, i.e. an expression level higher than an average expression level observed for a given molecule species, e.g., tRNA. The term "low expression" relates to a low expression level, i.e. an expression level lower than an average expression level observed for a given molecule species, e.g., tRNA.

The term "effective amount" in relation to a suppressor tRNA or a DNA construct comprising a nucleic acid encoding a suppressor tRNA refers to an amount of the suppressor tRNA in a pharmaceutical preparation or composition, or an amount of the suppressor tRNA intracellularly produced from the DNA construct, that is sufficient to bring about a desired result, e.g., an increased formation of a functional protein, for example enzyme, compared to the production of the protein in the absence of the suppressor tRNA or DNA construct.

The term "one, some or all of the sup-tRNAs of the composition" refers to one or more of the sup-tRNAs present in the composition. In a composition containing no more than five sup-tRNAs, for example, this refers to one, two, three, four or all five of the sup-tRNAs in the composition, In a composition containing no more than six sup-tRNAs, for example, this refers to one, two, three, four, five or all six of the sup-tRNAs in the composition. The term "one, some or all" in relation to any other entity, e.g., a number of nucleic acids encoding a sup-tRNA, for example, analogously refers to one or more of these entities, for example to one, two, three, four, five or, if applicable, six nucleic acids.

The term "pharmaceutically acceptable carrier" refers to a carrier that is suitable for the preparation of a pharmaceutical composition, and that is generally non-toxic and biologically safe, in particular for veterinary and/or human pharmaceutical use. An example of a simple carrier is a buffer solution or a physiologic salt solution. Other examples are nanoparticles like liposomes, Lipid-Nanoparticles (LNPs), or exosomes.

The term "pharmaceutically acceptable excipient" relates to any substance other than the active pharmaceutical ingredient (API) intentionally included in a pharmaceutical composition. An excipient is preferably, but not necessarily, an essentially pharmacologically inert (pharmacologically inactive) and non-toxic substance. Excipients can, for example, be included in a pharmaceutical composition in order to protect, support, or enhance stability, bioavailability, or patient acceptability of the API or pharmaceutical composition, to aid in the processing of the API during its manufacture, to assist in the effectiveness and/or delivery of the pharmaceutical composition, or to assist in maintaining the integrity of the pharmaceutical composition during storage. Examples are plasticizers, adjuvants, anti adherents, binders, disintegrants, glidants, lubricants, humectants, buffers, adhesives, thickeners, preservatives, coatings, fillers, diluents, flavoring agents, colorants, sweeteners etc.

In a preferred embodiment of the pharmaceutical composition according to the invention, the at least five suppressor transfer RNAs are selected from the group of suppressor transfer RNAs consisting of: tRNA-Arg-UR¹R², tRNA-Ser-UR¹R², tRNA-Gln-UR¹R², tRNA-Cys-UR¹R², tRNA-Glu-UR¹R², tRNA-Gly-UR¹R², tRNA-Tyr-UR¹R², tRNA-Lys-UR¹R², tRNA-Trp-UR¹R², and tRNA-Leu-UR¹R², wherein R¹ and R² are, independently from each other, A or G, with the proviso that R¹ and R² are not both G, preferably selected from the group of suppressor transfer RNAs consisting of: tRNA-Arg-UGA, tRNA-Ser-UAG, tRNA-Ser-UAA, tRNA-Ser-UGA, tRNA-Gln-UAA, tRNA-Gln-UAG, tRNA-Cys-UGA, tRNA-Glu-UAG, tRNA-Glu-UAA, tRNA-Gly-UGA, tRNA-Tyr-UAG, tRNA-Tyr-UAA, tRNA-Lys-UAA, tRNA-Lys-UAG, tRNA-Trp-UGA, tRNA-Trp-UAG, tRNA-Leu-UGA, tRNA-Leu-UAA, and tRNA-Leu-UAG.

The above designations for suppressor tRNA indicate the cognate amino acid and the targeted PTC according to the scheme tRNA-aa-PTC ("aa" = cognate amino acid). UR¹R² is one of the stop codons UGA, UAG or UAA. It should be noted here, that the codons (on mRNA level to which each tRNA pairs with its anticodon) are given here instead of the corresponding anticodons for a better understanding, although each suppressor tRNA carries, of course, the corresponding anticodon in its anticodon loop.

The group of suppressor transfer RNAs from which at least five suppressor transfer RNAs are selected for the pharmaceutical composition according to the invention consists of suppressor transfer RNAs having, as their cognate amino acid, the following ten amino acids: tryptophan (Trp), tyrosine (Tyr), cysteine (Cys), glutamic acid (Glu), lysine (Lys), glutamine (Gln), serine (Ser), leucine (Leu), arginine (Arg), and glycine (Gly). The amino acids mentioned are particularly vulnerable to PTC conversion. Each of the suppressor transfer RNAs carries an anticodon being configured to bind to one of the stop codons UGA, UAG or UAA.

The pharmaceutical composition of the invention may comprise more than one suppressor transfer RNAs which base-pairs to the same stop codon, i.e., carries the same anticodon. Preferably, however, the pharmaceutical composition of the invention comprises a maximum of three suppressor transfer RNAs base-pairing to the same stop codon in order to minimize the effect of amino acid misincorporation. However, if the composition contains more than one suppressor transfer RNA base-pairing to the same stop codon, the suppressor transfer RNAs base-pairing to the same stop codon differ in their cognate amino acid. Further, if the pharmaceutical composition of the invention contains more than one suppressor transfer RNA carrying the same anticodon, these suppressor transfer RNA preferably have tRNA bodies, or parts thereof, from non-cognate tRNAs only in order to decrease the interference for reading the same PTC. Such non-cognate tRNAs are, for example, tRNAs the bodies or body parts of which are, for example, those of naturally occurring non-cognate tRNAs that may or may not be modified (see below). This means that the pharmaceutical composition would not contain two tRNAs having the same anticodon, which bodies or body parts are, however, from naturally occurring near-cognate tRNAs that could possibly bind to the same sense codon, if carrying their natural anticodon. For example, a first suppressor tRNA targeting a specific PTC and comprising the body or body part of tRNA^{Arg} pairing to an AGA codon, or a tRNA^{Arg} body or body part modified as described below, should not be combined with a suppressor tRNA comprising a (optionally modified) body or body part of tRNA^{Ser} pairing to an AGU codon.

Further, if two or three suppressor tRNAs base-pairing to the same stop codon, i.e., carrying the same anticodon, are present in the pharmaceutical composition of the invention, these suppressor tRNAs are preferably present in different proportions, preferably in a proportion mirroring the PTC signature of the disease-associated gene.

In a particularly preferred embodiment the pharmaceutical composition of the invention, the pharmaceutical composition comprises five or six suppressor transfer RNAs, more preferably no more than five or six suppressor transfer RNAs. Particularly preferred, the pharmaceutical composition of the invention comprises the following suppressor tRNAs:
a) tRNA-Arg-UGA, tRNA-Ser-UAG, tRNA-Ser-UAA, tRNA-Gln-UAA, and tRNA-Cys-UGA; or
b) tRNA-Glu-UAG, tRNA-Glu-UAA, tRNA-Ser-UGA, tRNA-Gly-UGA, tRNA-Tyr-UAG, and tRNA-Lys-UAA; or
c) tRNA-Trp-UGA, tRNA-Trp-UAG, tRNA-Gln-UAG, tRNA-Lys-UAG, and tRNA-Tyr-UAA.

The three compositions according to features a) to c) above include five (a and c) or six (b) suppressor tRNAs, and preferably no more than the five or six suppressor tRNAs mentioned. It is to be noted that none of the suppressor tRNAs in one of the three above compositions is present in any of the other two compositions. The suppressor tRNAs in each composition differ in their combination of cognate amino acid and PTC.

In a further preferred embodiment of the pharmaceutical composition according to the invention, the proportion of each suppressor transfer RNA within the composition is adapted to the frequency of PTC mutations in a given gene of interest, such that a first suppressor transfer RNA configured to suppress a PTC with the highest frequency is present in the composition with the largest proportion, and a second, third, fourth and further suppressor transfer RNA configured to suppress a PTC with a lower frequency is present in the composition with a corresponding lower proportion, depending on the frequency of the targeted PTC in the gene of interest. In this embodiment, the proportion of the suppressor transfer RNAs within the pharmaceutical composition is determined based on the PTC pattern of a particular disease to be treated with the pharmaceutical composition, preferably based on the PTC pattern observed in a patient to be treated. The PTC pattern can be determined by determining the frequency of PTCs in a given disease and/or patient. Such patterns are known in the prior art or can be determined with routine experimentation.

The pharmaceutical composition can be adapted, for example in terms of the type and proportion of the suppressor transfer RNAs present in the composition, to the particular disease and/or patient. Those suppressor transfer RNAs present in the composition that target the PTCs of interest, i.e., the PTCs determined as normally or often present in a particular disease and/or present in a particular patient, are present in a higher proportion than those PTCs known to be not or rarely present in the respective disease or patient. Particularly preferred, the proportion of the suppressor transfer RNAs present in the composition is individually adapted to the PTC pattern of a given disease or patient to be treated.

Preferably, the proportion of the suppressor transfer RNAs present in the composition targeting a PTC in a gene of interest having a comparatively high frequency, preferably a frequency of at least 0,5%, more preferably at least 1%, 2%, 3%, 4% or 5% (see, e.g., Fujikura, K. Premature termination codons in modern human genomes. Sci Rep 6, 22468 (2016). https://doi.org/10.1038/srep22468), as known in the art or determined for the disease and/or patient, is 95% by weight of the entirety of the suppressor transfer RNAs present in the composition. As an example, if a PTC-related disease is known to have a PTC pattern of three frequently occurring PTCs, the composition would be configured to include three sup-tRNAs targeting those three PTC in a proportion of ≥ 95 %, for example, in a proportion of 60 % of the first sup-tRNA, 25 % of the second sup-tRNA, and 15 % of the third sup-tRNA. The other (e.g. two or three) sup-tRNA would be present in an amount such that their proportion would, in sum, be ≤ 5 % by weight. As another example, a sup-tRNA targeting a frequently occurring PTC may be present in the composition in a proportion of ≥ 95 %, whereas the other sup-tRNA would be present in a total proportion of ≤ 5 %.

The total amount of the sup-tRNAs present in the pharmaceutical composition of the invention is adjusted with a view to maximum efficiency with no or low toxicity. For example, a pharmaceutical composition according to the invention may include a maximum of 250 ng per 10⁴ cells, preferably a maximum of 240, 230, 220, 210, 205 or 200 ng per 10⁴ cells sup-tRNAs (see below).

Any of the supp-tRNAs in the pharmaceutical composition may be a naturally occurring tRNA, or an engineered, for example microbiologically engineered, tRNA. Preferably the tRNA is engineered, for example, in that the tRNA has an altered anticodon, e.g., an anticodon base-pairing to a stop codon in an mRNA, or has a modified D arm, Anticodon arm, T arm or acceptor stem.

A part of, e.g. one, two or three, or all of the sup-tRNAs present in the pharmaceutical composition of the invention can comprise a B box comprising the sequence H₅₄U₅₅C₅₆G₅₇A₅₈N₅₉U₆₀, preferably, U₅₄U₅₅C₅₆G₅₇A₅₈N₅₉U₆₀, the index representing the positions of the nucleotides in the transfer RNA, the position numbering following transfer RNA numbering convention. N stands for any nucleotide (A, C, G or U), H stands for A, C, or T/U. Nucleotides with number 54-60 are, in the mature tRNA, arranged within the T arm of the mature tRNA, and form the T loop. A tRNA comprising a B box having the above sequence is transcribed more strongly than a tRNA lacking such a B box. The tRNA may be a natural or synthetic (engineered) transfer RNA.

In preferred embodiments of the pharmaceutical composition of the invention, one, some or all of the sup-tRNAs may be structurally modified in that the tRNA has a structurally modified D arm, anticodon arm, variable loop and/or T arm. "Structurally modified" means that the mature tRNA contains an anticodon arm, and/or a variable loop and/or a T-arm and/or a D arm that has been modified, i.e. has a nucleotide sequence, for example of the stem portion or the loop portion, that differs from the D arm and/or the anticodon arm and/or the variable loop and/or the T arm of a comparable naturally occurring tRNA. "Comparable" tRNAs are tRNA being aminoacylated by the same aminoacyl-tRNA synthetases. Particular preferred, the encoded tRNA contains, in its mature form, a D arm, an anticodon arm and/or a variable loop and/or a T arm that does not occur in natural tRNAs.

The anticodon arm of one, some or all of the sup-tRNAs in the composition may, for example, have one of the following general structures with a modified stem portion:
5'-GCAGG-AC-Loop-CCUGU-3'
5'-UUGGG-AC-Loop-CUCAA-3'
5'-UUGGA-AC-Loop-UUCAA-3'
5'-AUGGU-AC-Loop-ACCAU-3'
5 '-GCGGA-AC-Loop-UCCGC-3'
5 '-GCGGU-AC-Loop-ACCGC-3'
5 '-GGCGG-AC-Loop-CCGCC-3'
5 '-GGCGC-AC-Loop-GCGCC-3'
5'-UUGGG-AC-loop-CCCAA-3'
5'-CUGGA-AC-loop-UCCAG-3'
5 '-CCGGA-AC-loop-UCCGG-3'
5'-GCUGC-AC-loop-GCAGU-3'

In preferred embodiments of the pharmaceutical composition of the invention, the one, some or all of the sup-tRNAs comprises, for example, an anticodon (AC) arm of one of the following sequences:

| | |
|---|---|
| GCAGGNNNNNNNCCUGU | (SEQ ID NO: 1) |
| UUGGGNNNNNNNCUCAA | (SEQ ID NO: 2) |
| UUGGANNNNNNNUUCAA | (SEQ ID NO: 3) |
| AUGGUNNNNNNNACCAU | (SEQ ID NO: 4) |
| GCGGANNNNNNNUCCGC | (SEQ ID NO: 5) |
| GCGGUNNNNNNNACCGC | (SEQ ID NO: 6) |
| GGCGGNNNNNNNCCGCC | (SEQ ID NO: 7) |
| GGCGCNNNNNNNGCGCC | (SEQ ID NO: 8) |

The underlined N's represent a stop anticodon. N = A, C, G or U, or any modified base.

In further preferred embodiments of the pharmaceutical composition of the invention, the one, some or all of the sup-tRNAs comprises, for example, an anticodon (AC) arm of one of the following sequences:

| | |
|---|---|
| GCAGGCUNNNAACCUGU | (SEQ ID NO: 9) |
| UUGGGCUNNNAACUCAA | (SEQ ID NO: 10) |
| UUGGACUNNNAAUUCAA | (SEQ ID NO: 11) |
| AUGGUCUNNNAAACCAU | (SEQ ID NO: 12) |
| GCGGACUNNNAAUCCGC | (SEQ ID NO: 13) |
| GCGGUCUNNNAAACCGC | (SEQ ID NO: 14) |
| GGCGGCUNNNAACCGCC | (SEQ ID NO: 15) |
| GGCGCCUNNNAAGCGCC | (SEQ ID NO: 16) |

The underlined N's represent a stop anticodon. N = A, C, G or U, or any modified base.

In a further preferred embodiment of the pharmaceutical composition of the invention, one, some or all of the sup-tRNAs comprise, in combination with one of the AC arms mentioned above, or alone, i.e., not in combination with one of the AC arms mentioned above, a variable loop (V loop) having one of the following sequences:
UGGGGUUUCCCC (SEQ ID NO: 17)
AGGGGAAACCCC (SEQ ID NO: 18)

In a further preferred embodiment of the pharmaceutical composition of the invention, one, some or all of the sup-tRNAs of the composition comprise, in combination with one of the AC arms described above and/or in combination with one of the V loops described above, or alone, i.e. not in combination with one of the AC arms mentioned above and/or not in combination with one of the V loops mentioned above, a T arm having the following general sequence:
GCGGG-T-Loop-CCCGU
ACGGG-T-Loop-CCCGU
GUAGG-T-Loop-CCCAU
GUCGG-T-Loop-CCCGU
GGCGG-T-Loop-CCGGU
GCAGG-T-Loop-CCCGU
GCCGG-T-Loop-CCGGU

Preferably, the T-Loop (positions 54-60 according to tRNA numbering convention) has the sequence HUCGANU, H being A, C, or U, i.e., CUCGANU, AUCGANU or UUCGANU, further preferred UUCGANU, for example UUCGAAU or UUCGAGU, preferably UUCGAGU.

In preferred embodiments of the pharmaceutical composition of the invention, one, some or all of the sup-tRNAs of the composition comprise, for example, a T arm of one of the following sequences

| | |
|---|---|
| GCGGG*UUCGAAU*CCCGU | (SEQ ID NO: 19) |
| ACGGG*UUCGAAU*CCCGU | (SEQ ID NO: 20) |
| GCGGG*UUCGAGU*CCCGU | (SEQ ID NO: 21) |
| ACGGG*UUCGAGU*CCCGU | (SEQ ID NO: 22) |
| GUAGG*UUCGAGU*CCCAU | (SEQ ID NO: 23) |
| GUCGG*UUCGAGU*CCGAU | (SEQ ID NO: 24) |
| GCCGG*UUCGAGU*CCGGU | (SEQ ID NO: 25) |
| GCAGG*UUCGAGU*CCCGU | (SEQ ID NO: 26) |
| GCCGG*UUCGAGU*CCGGU | (SEQ ID NO: 27) |

The loop part is in italics. The T arm has a stem of five base pairs.

In a further preferred embodiment of the pharmaceutical composition of the invention, one, some or all of the sup-tRNAs of the invention comprise, in combination with one of the AC arms described above and/or in combination with one of the V loops described above and/or in combination with one of the T arms described above, or alone, i.e. not in combination with one of the AC arms mentioned above and/or not in combination with one of the V loops mentioned above and/or not in combination with one of the T arms described above, an acceptor stem having the following general sequence:
5'-GGCUCUG-rest tRNA-CAGAGUC-3'
5'-GGCGCUG-rest tRNA-CAGCGUC-3'
5'-GGCGCGG-rest tRNA-CGGCGUC-3'

The term "rest tRNA" refers to the part of the sup-tRNA between the 5' and 3' portions of the acceptor stem, and includes the D arm, V loops and T arm.

Any uracil nucleotide (U) mentioned here in relation to a tRNA or part of a tRNA, in particular in the above sequences of SEQ ID NOs: 1-27, is represented as a T (thymine) nucleotide in the sequence listing according to ST.26.

As mentioned above, for any given sup-tRNA in the pharmaceutical composition of the invention, the anticodon arm or at least the anticodon stem, the V loop, the T arm, or at least the T stem, and the acceptor stem can be independently selected from the anticodon arms/stems, V loops, T arms/stems and acceptor stems mentioned above. The sup-RNA may thus only include one of the anticodon arms/stems, V loops, T arms/stems or acceptor stems mentioned above, for example only an anticodon arm with the sequence of SEQ ID NO: 10, or any combination thereof, for example an anticodon arm with the sequence of SEQ ID NO: 15 and a T arm having the sequence of SEQ ID NO: 21, or an anticodon arm with the sequence of SEQ ID NO: 10 and an acceptor stem as described above.

In preferred embodiments, the pharmaceutical composition of the invention includes at least one tRNA, preferably at least two, three, four or five tRNAs comprising:
a) an anticodon arm having or comprising one of the sequences of SEQ ID NO: 1-16, and/or
b) a variable loop having or comprising one of the sequences of SEQ ID NO: 17-18, and/or
c) a T arm having or comprising one of the sequences according to SEQ ID NO: 19-27, and/or
d) an acceptor stem having the structure 5'-GGCUCUG-rest tRNA-CAGAGUC-3' or 5'-GGCGCUG-rest tRNA-CAGCGUC-3'. Particularly preferred, all of the tRNAs of the pharmaceutical composition have an anticodon arm, and/or variable loop and/or T arm selected from one of the respective sequences above.

The skilled person is aware of the fact that a tRNA is aminoacylated with a specific amino acid by a specific aminoacyl tRNA synthetase (aaRS), and that the aaRS is able to recognize its cognate tRNA through unique identity elements at the acceptor stem and/or anticodon loop of the tRNA. In order to include in the DNA construct of the invention a tRNA gene of a tRNA which is, in its mature form, loaded with its cognate amino acid in vivo, the skilled person will design the encoded tRNA with suitable unique identity elements.

The pharmaceutical composition of the invention may include any pharmaceutically acceptable excipient(s).

In a second aspect, the invention provides a synthetic DNA construct comprising a) at least five nucleic acids each encoding a different suppressor transfer RNA and b) DNA regulatory elements functionally linked to the nucleic acids in such a manner, that at least one of the five nucleic acids can be expressed independent of the other nucleic acids in the construct.

The synthetic DNA construct of the invention comprises at least five, for example five or six, nucleic acids each encoding a different transfer RNA, preferably one of the transfer RNAs described above in the context of the pharmaceutical composition of the first aspect of the invention. This does not exclude, however, that the synthetic DNA construct of the invention may comprise more than one of the nucleic acids selected from the group consisting of the five or six nucleic acids encoding different transfer RNA. In order to enhance expression of one or more of the nucleic acids, two or more copies of one, two or more of the five or six nucleic acids may be arranged within the synthetic DNA construct of the invention. Further, the DNA construct of the invention comprises DNA regulatory elements functionally linked to the nucleic acids in such a manner, that at least one of the five nucleic acids encoding the tRNAs can be expressed independent of the other nucleic acids in the construct. The construct can, for example, be configured to include a first DNA regulatory element for the control of expression a first nucleic acid encoding a first sup-tRNA, and a common second DNA regulatory element for the control of expression of the other nucleic acids encoding the other sup-tRNAs. This allows for the individual expression, for example at a higher level, of at least one the sup-tRNA. The construct preferably comprises at least two, three, four or five DNA regulatory elements for the individual control of expression of the nucleic acids encoding the sup-tRNA functionally liked to the DNA regulatory elements. Preferably, the DNA construct is configured to include a DNA regulatory element for each of the nucleic acids encoding the different sup-tRNAs, such that an individual control of expression, for example the expression level, of the sup-tRNAs is possible. The construct can, for example, be configured in a manner that, in the context of a living cell, e.g., human cell, into which the construct has been introduced, three of the sup-tRNAs are expressed, in relation to the entirety of tRNAs expressed from the construct, with a percentage of ≥ 95 %, for example, in a percentage of 60 % of the first sup-tRNA, 25 % of the second sup-tRNA, and 15 % of the third sup-tRNA, whereas the other (e.g. two or three) sup-tRNA would be expressed in an amount such that their percentage would, in sum, be ≤ 5 % by weight. As another example, a DNA construct of the invention can be configured to cause suppression of a single sup-tRNA targeting a frequently occurring PTC in a proportion of ≥ 95 %, whereas the other sup-tRNAs would be expressed in a total proportion of ≤ 5 %.

The DNA construct of the invention can, for example, be configured as a gene delivery vehicle (GDV) for the delivery of suppressor tRNA into cells, in particular mammalian cells, e.g., human cells.

The sup-tRNAs encoded by the nucleic acids are arranged in the synthetic DNA construct, e.g., a synthetic vector, in a transcribable form, i.e., in a form that they are transcribed under natural conditions once introduced in a living mammalian cell, e.g., human cell, such that the encoded tRNAs are produced in the cell, preferably using the natural transcription machinery of the cell. The tRNAs may be arranged in a manner that it can be conditionally transcribed, i.e., depending on specific conditions in the cell. Preferably, the sup-tRNAs encoded by the nucleic acids are arranged in the synthetic DNA construct in such a manner that they can be expressed independently from each other, and are, for example, each functionally linked to different regulatory elements determining their respective expression level.

Preferably, the synthetic DNA construct of the invention, comprises at least one nucleic acid encoding a tRNA, the tRNA comprising:
a) an anticodon arm having or comprising one of the sequences of SEQ ID NO: 1-16, and/or
b) a variable loop having or comprising one of the sequences of SEQ ID NO: 17-18, and/or
c) a T arm having or comprising one of the sequences according to SEQ ID NO: 19-27, and/or
d) an acceptor stem having the structure 5'-GGCUCUG-rest tRNA-CAGAGUC-3' or 5'-GGCGCUG-rest tRNA-CAGCGUC-3'. Particularly preferred, at least two, three, four, five or all of the encoded tRNAs have an anticodon arm, and/or variable loop and/or T arm selected from one of the respective sequences above. In order to avoid any misunderstanding it should be noted here, that the sequences mentioned above, relating to tRNA parts, e.g., to an anticodon arm, a variable loop or a T arm, are, on DNA level, identical to the sequences presented above and/or having the sequence identification numbers given above, however, with U replaced by T.

In a third aspect the invention relates to a pharmaceutical composition comprising a) at least five, preferably five or six, different synthetic DNA constructs, wherein each of the synthetic DNA constructs comprises a nucleic acid encoding a different suppressor transfer RNA and a DNA regulatory element functionally linked to the nucleic acid in such a manner, that at least one of the five nucleic acids in the pharmaceutical composition can be expressed at an expression level different from the expression level of the other nucleic acids in the composition, or wherein at least one of the at least five, preferably five or six, different synthetic DNA constructs is present in the pharmaceutical composition in a higher copy number than the other synthetic DNA constructs, and b) a pharmaceutically acceptable carrier.

Any of the DNA constructs in the pharmaceutical composition of the invention can, for example, be configured as a gene delivery vehicle (GDV) for the delivery of suppressor tRNA into cells, in particular mammalian cells, e.g., human cells.

The pharmaceutical composition according to this third aspect of the invention comprises at least five, for example five or six, different synthetic DNA constructs, each of the synthetic DNA constructs comprising a nucleic acid encoding a different suppressor transfer RNA. Each of the synthetic DNA constructs of the pharmaceutical composition preferably comprises a single nucleic acid encoding a suppressor transfer RNA that is different from the suppressor transfer RNA encoded by the nucleic acids included in the other synthetic DNA constructs. Each of the synthetic DNA constructs is, for example, configured in a manner that the nucleic acid encoding the suppressor transfer RNA can be individually expressed from the synthetic DNA construct, such that, for example, a first suppressor transfer RNA is, when introduced into a living cell, e.g., human cell, expressed at a first expression level, which is different from all or at least part the other suppressor transfer RNAs. Alternatively, or additionally, at least one of the at least five, for example five or six, different synthetic DNA constructs is present in the pharmaceutical composition in a higher copy number than the other synthetic DNA constructs, in order to allow for a higher expression level of those tRNA that are included in the DNA constructs being present in a higher copy number. Further, it is also possible, of course, to include more than one copy of a nucleic acid encoding the same tRNA in the same DNA construct or in different synthetic DNA constructs.

The pharmaceutical composition of the invention according to this third aspect can include any pharmaceutically acceptable excipient(s).

The expression level of the nucleic acids, be it in the single synthetic DNA construct of the invention according to the second aspect of the invention, or be it in the at least five synthetic DNA constructs combined in the pharmaceutical composition according to the third aspect of the invention, can, for example, be controlled by upstream 5' leader sequences functionally linked the nucleic acid(s) encoding the sup-tRNA. Another option is to arrange multiple copies of at least one of the at least five nucleic acids encoding a suppressor transfer RNA within a single synthetic DNA or multiple copies of the same synthetic DNA construct in a pharmaceutical composition. The number of the copies of the nucleic acid(s) or DNA construct(s) can be adapted to the PTC signature of the disease to be treated. The following more detailed description of suitable 5' leader sequences for expression control relates to both the synthetic DNA construct of the second aspect of the invention and the synthetic DNA constructs in the pharmaceutical composition according to the third aspect of the invention. It should thus be noted, that, even if in the following 5' leader sequences are described only in the context of the single DNA construct of the invention according to the second aspect of the invention, or in the context of the synthetic DNA constructs included in the pharmaceutical composition according to the third aspect of the invention, the description relates to both aspects.

The sequence motifs in the 5' leader sequence described below cause the tRNA(s) arranged downstream from the 5' leader sequence to be more strongly (at a higher level) or more weakly (at a lower level) transcribed than in the absence of these motifs.

In a preferred embodiment of the synthetic DNA construct of the second aspect of the invention, or of the pharmaceutical composition according to the third aspect of the invention, the at least five nucleic acids each encode a different suppressor transfer RNA, wherein at least one, two, three, four, five or all of the nucleic acids are each, independently from each other, functionally linked to a 5' leader sequence comprising:
a) at least one sequence motif selected from the group consisting of the sequence motifs TGACCTAAGTGTAAAGT, I_{H}, (SEQ ID NO: 28), TGAGATTTCCTTCAGGTT, II_{H}, (SEQ ID NO: 29), TATATAGTTCTGTATGAGACCACTCTTTCCC, III_{H}, (SEQ ID NO: 30), ACCATAAACGTGAAATG, I_{L}, (SEQ ID NO: 31), TCTTTGGATTTGGGAATC, II_{L}, (SEQ ID NO: 32, and TTATAAGTTCTGTATGAGACCACTCTTTCCC, III_{L}, (SEQ ID NO: 33); or
b) one sequence motif selected from the group consisting of the sequence motifs VNANANTVHANANNTTNNNATRANATTCNGDGVGNAANATABTNCTVGND, 50nt_{H}, (SEQ ID NO: 34) and GCNGGDGGCGNGTTCBBCTGTNVNAGCNTNCGDNGNCGTTTNNCNTCGGT, 50nt_{L}, (SEQ ID NO: 35); or
c) at least one sequence motif selected from the group consisting of the sequence motifs GAAATGCCTT, 10nt_{H1}, (SEQ ID NO: 36), GTGGGAACTA, 10nt_{H2}, (SEQ ID NO: 37), and GTGTTGCTTG, 10nt_{H3}, (SEQ ID NO: 38)
and wherein at least one of the nucleic acids encoding a suppressor transfer RNA has a 5' leader sequence different from the other nucleic acids encoding a suppressor transfer RNA.

Besides a tRNA gene, the single synthetic DNA construct of the invention or the synthetic DNA constructs in the pharmaceutical composition comprise a DNA regulatory element being or comprising a 5' leader sequence functionally linked to the nucleic acid encoding the tRNA (tRNA gene), wherein the 5' leader sequence comprises sequence motifs for the binding of the transcription factor TFIIIB. The sequence motifs have promoter activity that can be included in a 5' leader sequence of a tRNA encoded downstream of the 5' leader sequence. Suitable selection and, as the case may be, combination of the sequence motifs enables control of the transcription and thus the expression level of the downstream tRNA gene. The tRNA encoded by the nucleic acid in the construct(s) may, for example, be an engineered tRNA, having, for example, a modified anticodon being able to base-pair with a stop codon on an mRNA, and/or a modified T arm comprising a B box sequence motif, as described above. The B box sequence may, for example, be a sequence enhancing the binding of the transcription factor TFIIIC to the tRNA gene.

The invention provides, for insertion into one or more of the constructs described herein, in a first embodiment, three sequence motifs, designated I_{H} (TGACCTAAGTGTAAAGT, SEQ ID NO: 28), II_{H} (TGAGATTTCCTTCAGGTT, SEQ ID NO: 29) and III_{H} (TATATAGTTCTGTATGAGACCACTCTTTCCC, SEQ ID NO: 30), promoting high expression of the tRNA, and three sequence motifs, I_{L} (ACCATAAACGTGAAATG, SEQ ID NO: 31), II_{L} (TCTTTGGATTTGGGAATC, SEQ ID NO: 32) and III_{L} (TTATAAGTTCTGTATGAGACCACTCTTTCCC, SEQ ID NO: 33), promoting low expression of the tRNA, when present in the 5' leader sequence of the tRNA encoded in a nucleic acid. The sequence motifs can be included in a 5' leader sequence of an associated tRNA gene in order to control the level of expression of the tRNA.

Preferably, the sequence motifs I_{H} and I_{L} are not both present in the same 5' leader sequence. The same applies to the sequence motifs II_{H} and II_{L} and to the sequence motifs III_{H} and III_{L}. Therefore, it is thus preferred that
i. the 5' leader sequence does not comprise the sequence motif In if it comprises the sequence motif I_{L}, and vice versa,
ii. the 5' leader sequence does not comprise the sequence motif II_{H} if it comprises the sequence motif II_{L}, and vice versa, and that
iii. the 5' leader sequence does not comprise the sequence motif III_{H} if it comprises the sequence motif III_{L}, and vice versa.

Preferably, the sequence motifs I_{L}, II_{L}, III_{L}, I_{H}, II_{H} or III_{H}, if present, are arranged upstream of the tRNA encoding nucleic acid at specific positions. In particular, the sequence motifs I_{L} and I_{H} are, if present, arranged in such a manner that they occupy positions -66 to -50, the positions relating to the region upstream of the nucleic acid encoding the tRNA . The sequence motifs II_{L} and II_{H} are, if present, arranged in such a manner that they occupy positions -49 to -32, and the sequence motifs III_{L} and III_{H} are, if present, arranged in such a manner that they occupy positions -31 to -1. The sequence motifs are thus preferably arranged in such a manner that
i. if the 5' leader sequence comprises the sequence motif I_{H} or I_{L}, the sequence motif I_{H} or I_{L} has, in 5'-3' direction, the positions -66 to -50,
ii. if the 5' leader sequence comprises the sequence motif II_{H} or II_{L}, the sequence motif II_{H} or IIL has, in 5'-3' direction, the positions -49 to -32, and
iii. if the 5' leader sequence comprises the sequence motif III_{H} or III_{L}, the sequence motif III_{H} or III_{L} has, in 5'-3' direction, the positions -31 to -1.

Further preferred, the 5' leader sequence comprises at least two sequence motifs selected from the sequence motifs I_{L}, II_{L}, III_{L}, I_{H}, II_{H} or III_{H}, wherein the at least two sequence motifs are arranged, in 5'-3' direction, in the order I_{H} - II_{H}, II_{H} - III_{H}, I_{H} - III_{H}, I_{L} - II_{L}, II_{L} - III_{L}, I_{L} - III_{L}, I_{H} - II_{L}, I_{H} - III_{L}, II_{H} - III_{L}, I_{L} - II_{H}, II_{L} - III_{H}, or I_{L} - III_{H}.

As mentioned above, the sequence motifs are preferably arranged such that they take the positions mentioned, i.e., positions -66 to -50 for sequence motifs I_{L} and I_{H}, positions -49 to -32 for sequence motifs II_{L} and II_{H}, and positions -31 to -1 for sequence motifs IIIL and III_{H}.

Further preferred, the 5' leader sequence comprises three sequence motifs selected from the sequence motifs I_{L}, II_{L}, III_{L}, I_{H}, II_{H} or III_{H}. Preferably, the sequence motifs are arranged in the following order, in 5'-3' direction: I_{L} - II_{L} - IIIL, I_{H} - II_{H} - III_{H}, I_{H} - II_{L} - III_{L}, I_{L} - II_{H} - III_{L}, I_{L} - II_{L} - III_{H}, I_{H} - II_{H} - III_{L}, I_{H} - II_{L} - III_{H}, or I_{L} - II_{H} - III_{H}. Preferably, the sequence motifs are arranged directly one after the other, i.e., without nucleotides or linkers in between. Again, the sequence motifs preferably have the positions mentioned above.

In preferred embodiments of the synthetic DNA construct according to the second aspect of the invention or the pharmaceutical composition according to the third aspect of the invention of the invention, the 5' leader sequence may have one of the sequences according to SEQ ID NOs: 39-64, according to the following list:
P1 (I_{H}-II_{H}-III_{H}, SEQ ID NO: 39): 
TGACCTAAGTGTAAAGTTGAGATTTCCTTCAGGTTTATATAGTTCTGTATGAGACCA
CTCTTTCCC
P2 (I_{L}-II_{L}-III_{L}, SEQ ID NO: 40): 
ACCATAAACGTGAAATGTCTTTGGATTTGGGAATCTTATAAGTTCTGTATGAGACC
ACTCTTTCCC
P3 (I_{H}, SEQ ID NO: 41): 
TGACCTAAGTGTAAAGTNNNNNNNNNNNNNNNNNNNNNNNNNNNNNNNNNNNNNNNN
NNNNNNNNNNNNN
P4 (II_{H}, SEQ ID NO: 42): 
NNNNNNNNNNNNNNNNNNNNTGAGATTTCCTTCAGGTTNNNNNNNNNNNNNNNNNNNNNN
NNNNNNNNNNNNNN
P5 (III_{H}, SEQ ID NO: 43): 
NNNNNNNNNNNNNNNNNNNNNNNNNNNNNNNNNNTATATAGTTCTGTATGAGA
CCACTCTTTCCC
P6 (I_{H}-II_{H}, SEQ ID NO: 44): 
TGACCTAAGTGTAAAGTTGAGATTTCCTTCAGGTTNNNNNNNNNNNNNNNNNNNNN
NNNNNNNNNNNN
P7 (II_{H}-III_{H}, SEQ ID NO: 45): 
NNNNNNNNNNNNNNNNNNNNTGAGATTTCCTTCAGGTTTATATAGTTCTGTATGAGACC
ACTCTTTCCC
P8 (I_{H}-III_{H}, SEQ ID NO: 46): 
TGACCTAAGTGTAAAGTNNNNNNNNNNNNNNNNNNTATATAGTTCTGTATGAGAC
CACTCTTTCCC
P9 (I_{L}, SEQ ID NO: 47): 
ACCATAAACGTGAAATGNNNNNNNNNNNNNNNNNNNNNNNNNNNNNNNNNNNN
NNNNNNNNNNNN
P10 (II_{L}, SEQ ID NO: 48): 
NNNNNNNNNNNNNNNNNNNNTCTTGGATTTGGGAATCNNNNNNNNNNNNNNNNNNNNNNNNNNNN
NNNNNNNNNNNNNN
P11 (III_{L}, SEQ ID NO: 49): 
NNNNNNNNNNNNNNNNNNNNNNNNNNNNNNNNNNNNNTTATAAGTTCTGTATGAGA
CCACTCTTTCCC
P12 (I_{L}-II_{L}, SEQ ID NO: 50): 
ACCATAAACGTGAAATGTCTTTGGATTTGGGAATCNNNNNNNNNNNNNNNNNNNNN
NNNNNNNNNNNN
P13 (II_{L}-III_{L}, SEQ ID NO: 51): 
NNNNNNNNNNNNNNNNNNTCTTGGATTTGGAATCTTATAAGTTCTGTATGAGACC
ACTCTTTCCC
P14 (SEQ ID NO: 52): 
ACCATAAACGTGAAATGNNNNNNNNNNNNNNNNNNTTATAAGTTCTGTATGAGAC
CACTCTTTCCC
P15 (SEQ ID NO: 53): 
TGACCTAAGTGTAAAGTTCTTGGATTTGGGAATCNNNNNNNNNNNNNNNNNNNNNNNNNN
NNNNNNNNNNNN
P16 (SEQ ID NO: 54): 
TGACCTAAGTGTAAAGTNNNNNNNNNNNNNNNNTTATAAGTTCTGTATGAGAC
CACTCTTTCCC
P17 (SEQ ID NO: 55): 
NNNNNNNNNNNNNNNNNNNNTGAGATTTCCTTCAGGTTTATAAGTTCTGTATGAGACC
ACTCTTTCCC
P18 (SEQ ID NO: 56): 
ACCATAAACGTGAAATGTGAGATTTCCTTCAGGTTNNNNNNNNNNNNNNNNNNNN
NNNNNNNNNNNN
P19 (SEQ ID NO: 57): 
NNNNNNNNNNNNNNNNNNNNTCTTTGGATTTGGAATCTATATAGTTCTGTATGAGACC
ACTCTTTCCC
P20 (SEQ ID NO: 58): 
ACCATAAACGTGAAATGNNNNNNNNNNNNNNNNNNTATATAGTTCTGTATGAGAC
CACTCTTTCCC
P21 (SEQ ID NO: 59): 
TGACCTAAGTGTAAAGTTCTTTGGATTTGGGAATCTTATAAGTTCTGTATGAGACCA
CTCTTTCCC
P22 (SEQ ID NO: 60): 
ACCATAAACGTGAAATGTGAGATTTCCTTCAGGTTTTATAAGTTCTGTATGAGACC
ACTCTTTCCC
P23 (SEQ ID NO: 61): 
ACCATAAACGTGAAATGTCTTTGGATTTGGGAATCTATATAGTTCTGTATGAGACC
ACTCTTTCCC
P24 (SEQ ID NO: 62): 
TGACCTAAGTGTAAAGTTGAGATTTCCTTCAGGTTTTATAAGTTCTGTATGAGACCA
CTCTTTCCC
P25 (SEQ ID NO: 63): 
TGACCTAAGTGTAAAGTTCTTTGGATTTGGGAATCTATATAGTTCTGTATGAGACCA
CTCTTTCCC
P26 (SEQ ID NO: 64):

ACCATAAACGTGAAATGTGAGATTTCCTTCAGGTTTATATAGTTCTGTATGAGACC
ACTCTTTCCC

N stands for any nucleotide (A, G, C or T).

In a second embodiment of the synthetic DNA construct or the pharmaceutical composition of the invention, the 5' leader sequence comprises one sequence motif selected from the group consisting of the sequence motifs VNANANTVHANANNTTNNNATRANATTCNGDGVGNAANATABTNCTVGND, designated 50nt_{H}, (SEQ ID NO: 34) and GCNGGDGGCGNGTTCBBCTGTNVNAGCNTNCGDNGNCGTTTNNCNTCGGT, designated 50nt_{L}, (SEQ ID NO: 35). The abbreviations have the standard meaning as indicated above (see, e.g., ST.26 ver. 1.5).

The sequence motif 50nt_{H} promotes a higher expression of the downstream tRNA gene, and 50nt_{L} causes a lower expression of the downstream tRNA gene.

In a preferred embodiment of this second embodiment of the synthetic DNA construct or the pharmaceutical composition of the invention the 5' leader sequence does not comprise the sequence motif 50nt_{H} if it comprises the sequence motif 50nt_{L}, and vice versa.

Further preferred, the sequence motifs 50nt_{H} or 50nt_{L} have the positions -50 to -1, i.e., if the sequence of 50nt_{H} is present, it preferably takes, in 5'-3' direction, the positions -50 to -1, and if the sequence of 50nt_{L} is present, it takes, in 5'-3' direction, positions -50 to -1.

The sequence motif 50nt_{H} may have one of the following sequences:
P27 (50nt_{H1}, SEQ ID NO: 65) CCATGATCCCCCACTATTAAGGATATCCGGAGAGGATGCTACCTATCAGG
P28 (50nt_{H2}, SEQ ID NO: 66) AGACCAGCTGTATAGCCTCAGAATGATCCGTCCGGAAACCCTTACTAGGT
P29 (50nt_{H3}, SEQ ID NO: 67) GCATATTGCAGAACTTGTGAAGAGGCTTTATGCGCCACACACTGCAAGCA
P30 (50nt_{H4}, SEQ ID NO: 68) GCAAAAGCAAAAGCATTAAGTAGCTTTCTGGCATAAACATATTGCAGCTG
P31 (50nt_{H5}, SEQ ID NO: 69) GGTTGCGGTAAGCATTAGAGGGCTATCAGCAGCATCTTATCGCAGCGGAG
P32 50nt_{H6}, (SEQ ID NO: 70) CTGCAGTATAACCTTGAAGTACCATCACGGAGAGAAACAGTGCCATCTCA
P33 (50nt_{H7}, SEQ ID NO: 71) AACGACTACGTAGTGTTCTATAACAATCATGAGAAATTTTAGTTCTAGAT

The sequence motif 50nt_{L} may have one of the following sequences:
P34 (50nt_{L1}, SEQ ID NO: 72) GCTGGTGGCGAGTTCCGCTGTGCCAGCTTCCGTTGGCGTTTGCCATCGGT
P35 (50nt_{L2}, SEQ ID NO: 73) GCTGGTGGCGAGTTCCGCTGTGCCAGCTTCCGTTGGCGTTTGCCATCGGT
P36 (50nt_{L3}, SEQ ID NO: 74) GCTGGTGGCGAGTTCCGCTGTGCCAGCTTCCGTTGGCGTTTGCCATCGGT
P37 (50nt_{L4}, SEQ ID NO: 75) ATGCGGCCTTGTGTGGTGGCTCATTGTGGTGGGCCAGAAAACGCGTGCAA
P38 (50nt_{L5}, SEQ ID NO: 76) GCCAGAGGCGCTGGGGCCAGGAGGCGCAAGCCGGGCCCTGAAGCCGCCTC
P39 (50nt_{L6}, SEQ ID NO: 77) AAAGAAATTAGGAAATTCCTGTGGAAGCTGGCGCGTTGATGCACTTCGTC
P40 (50nt_{L7}, SEQ ID NO: 78) CAACAAACATTTTGCTTTTTTAAAATTGAAAGAACAACTGTTTTCCGGGT

In a third embodiment of the synthetic DNA construct or the pharmaceutical composition of the invention, the 5' leader sequence of the nucleic acid encoding a transfer RNA comprises at least one sequence motif selected from the sequence motifs GAAATGCCTT, designated 10nt_{H1}, (SEQ ID NO: 36), GTGGGAACTA, designated 10nt_{H2}, (SEQ ID NO: 37), and GTGTTGCTTG, designated 10nt_{H3}, (SEQ ID NO: 38). The sequence motifs, designated 10nt_{H1}, 10nt_{H2} and 10nt_{H3}, can advantageously be used to promote higher expression of the tRNA gene arranged downstream of the 5' leader sequence within the synthetic DNA construct. The motifs are preferably arranged within a region from -100 to -1 in relation to the tRNA gene.

Preferably, the 5' leader sequence comprises at least two sequence motifs selected from the sequence motifs 10nt_{H1}, 10nt_{H2} and 10nt_{H3}. Preferably, the at least two sequence motifs are:
i. GAAATGCCTT, 10nt_{H1}, (SEQ ID NO: 36) and GTGTTGCTTG, 10nt_{H3}, (SEQ ID NO: 38), wherein the sequence motifs are arranged, in 5'-3' direction, in the following order: GTGTTGCTTG (SEQ ID NO: 38) - GAAATGCCTT (SEQ ID NO: 36), i.e., 10nt_{H3} - 10nt_{H1}, or
ii. GAAATGCCTT, 10nt_{H1}, (SEQ ID NO: 36) and GTGGGAACTA, 10nt_{H2}, (SEQ ID NO: 37), wherein the sequence motifs are arranged, in 5'-3' direction, in the following order: GAAATGCCTT (SEQ ID NO: 36) - GTGGGAACTA (SEQ ID NO: 37), i.e., 10nt_{H1} - 10nt_{H2}, or
iii. GTGGGAACTA, 10nt_{H2}, (SEQ ID NO: 37), and GTGTTGCTTG, 10nt_{H3}, (SEQ ID NO: 38), wherein the sequence motifs are arranged, in 5'-3' direction, in the following order: GTGTTGCTTG (SEQ ID NO: 38) - GTGGGAACTA (SEQ ID NO: 37), i.e., 10nt_{H3} - 10nt_{H2}.

Further preferred, in this third embodiment of a synthetic DNA construct or pharmaceutical composition of the invention, the 5' leader sequence comprises all three sequence motifs GAAATGCCTT (SEQ ID NO: 36), 10nt_{H1}, GTGGGAACTA (SEQ ID NO: 37), 10nt_{H2}, and GTGTTGCTTG (SEQ ID NO: 38), 10nt_{H3}, preferably in the following order, in 5'-3' direction: GTGTTGCTTG (SEQ ID NO: 38) - GAAATGCCTT (SEQ ID NO: 36) - GTGGGAACTA (SEQ ID NO: 37), i.e., 10nt_{H3} - 10nt_{H1} - 10nt_{H2}.

The tRNA encoded by the nucleic acid may be a naturally occurring tRNA, or an engineered, for example microbiologically engineered, tRNA. Preferably the tRNA is engineered, for example, in that the tRNA has an altered anticodon, e.g., an anticodon base-pairing to a stop codon in an mRNA, or has a modified D arm, Anticodon arm, T arm or acceptor stem, as described above, for example.

In a preferred embodiment of any of the synthetic DNA constructs according to the invention, the nucleic acid encoding the tRNA comprises a B box comprising the sequence H₅₄T₅₅C₅₆G₅₇A₅₈N₅₉T₆₀, preferably, T₅₄T₅₅C₅₆G₅₇A₅₈N₅₉T₆₀, the index representing the positions of the nucleotides in the transfer RNA, the position numbering following transfer RNA numbering convention. N stands for any nucleotide (A, C, G or T), H stands for A, C, or T. Nucleotides with number 54-60 are, in the mature tRNA, arranged within the T arm of the mature tRNA, and form the T loop. A tRNA comprising a B box having the above sequence is transcribed more strongly than a tRNA lacking such a B box. The encoded tRNA may be a natural or synthetic (engineered) transfer RNA.

The nucleic acid encoding the transfer RNA may also contain one or more introns. These intron(s) may be natural occurring introns. In preferred embodiments the intron(s) is (are) inserted into a nucleic acid encoding a tRNA that does not have an intron naturally. However, the tRNA encoded by the nucleic acid can also be a tRNA comprising a tRNA body of a tRNA the gene or pre-tRNA of which naturally contains one or more introns, which are, however, preferably different from the introns inserted in the nucleic acid according to the invention. Inclusion of one or more introns allows, at least in some applications, for close to natural tRNA transcription with factors (enzymes) that co-transcriptionally modify tRNAs which in turn leads to more stable output as functional tRNA.

In preferred embodiments, the encoded tRNA may be structurally modified in that the tRNA has a structurally modified D arm, anticodon arm, variable loop and/or T arm. "Structurally modified" means that the mature tRNA contains an anticodon arm, and/or a variable loop and/or a T-arm and/or a D arm that has been modified, i.e. has a nucleotide sequence, for example of the stem portion or the loop portion, that differs from the D arm and/or the anticodon arm and/or the variable loop and/or the T arm of a comparable naturally occurring tRNA. "Comparable" tRNAs are tRNA being aminoacylated by the same aminoacyl-tRNA synthetases. Particular preferred, the encoded tRNA contains, in its mature form, a D arm, an anticodon arm and/or a variable loop and/or a T arm that does not occur in natural tRNAs.

In order to avoid unnecessary repetition, reference is made to the above description of structurally modified tRNA in the context of the pharmaceutical composition according to the first aspect of the invention. All of the tRNA and tRNA structures described above can also be included in any of the DNA constructs described in the context of the second or third aspect of the invention. Of course, any U nucleotide mentioned above on RNA level will have to be replaced with a T nucleotide on DNA level.

For any given tRNA encoded by any of the synthetic DNA constructs of the invention, the anticodon arm or at least the anticodon stem, the V loop, the T arm, or at least the T stem, and the acceptor stem can be independently selected from the anticodon arms/stems, V loops, T arms/stems and acceptor stems mentioned above. The encoded tRNA may thus only include one of the anticodon arms/stems, V loops, T arms/stems or acceptor stems mentioned above, for example only an anticodon arm with the sequence of SEQ ID NO: 10, or any combination thereof, for example an anticodon arm with the sequence of SEQ ID NO: 15 and a T arm having the sequence of SEQ ID NO: 21, or an anticodon arm with the sequence of SEQ ID NO: 10 and an acceptor stem as described above.

Any of the synthetic DNA constructs can, for example, be or incorporated in a synthetic vector, and thus be or incorporated in, for example, any suitable biological nucleic acid delivery vehicle, in particular a delivery vehicle suitable for delivery of a nucleic acid into a living mammalian cell, e.g., a human cell. Suitable vehicles include, for example, viral vectors such as adeno-associated virus (AAV)-based viral vectors, encapsulation in or coupling to nanoparticles, e.g., lipid nanoparticles, and others. Preferably, the vector is a viral vector. Viral vectors include, for example, adeno-associated (AAV) viruses, adenoviruses (e.g., AAV3, AAV8, or AAV9), retroviruses (see, for example, Bulcha, J.T., Wang, Y., Ma, H. et al. Viral vector platforms within the gene therapy landscape. Sig Transduct Target Ther 6, 53, 2021, doi: 10.1038/s41392-021-00487-6; Kotterman, M.A., Chalberg, T.W., Schaffer, D.V., 2015, Viral Vectors for Gene Therapy: Translational and Clinical Outlook, Annu. Rev. Biomed. Eng. 2015. 17:63-89, 10.1146/annurev-bioeng-071813-104938).

In a further aspect the invention relates to the pharmaceutical composition according to the first aspect of the invention, the synthetic DNA construct according to the second aspect of the invention, or the pharmaceutical composition according to the third aspect of the invention for use as a medicament. The pharmaceutical compositions or synthetic DNA constructs of the invention are especially useful for treating patients with a disease associated with a nonsense mutation, i.e. a premature termination codon (PTC), causing the absence of a functional protein or the dysfunction of a protein. Examples for such diseases, may advantageously be employed are Hurler syndrome (MPS I, ICD code E76.0), beta-thalassemia (ICD-10 code D56.1), neurofibromatosis type 1 (NF1, ICD-10 code Q85.0), Duchenne muscular dystrophy (DMD, ICD-10 code G71.0), Crohn's disease (CD, ICD-10 code K50), cystic fibrosis (CF, ICD-10 code E84), neuronal ceroid lipofuscinosis (NCL, ICD-10 code E75.4), spinal muscular atrophy (ICD-10 code G12.9) and Tay-Sachs disease (TSD, ICD-10 code E75.0). The DNA construct, e.g., vector, of the invention is also useful for treating patients with a disease which is at least partly caused by the sequestration of a tRNA leading to depletion of the cellular pool of the tRNA, e.g. Charcot-Marie-Tooth disease (CMT disease, ICD-10 code DG600).

In a further aspect, the invention relates to a kit comprising three pharmaceutical compositions according to the first aspect of the invention, with the proviso that none of the suppressor transfer RNAs present in one of the three pharmaceutical compositions is not also present in one of the two other pharmaceutical compositions. Preferably, each of the three pharmaceutical compositions does not contain more than five or six different suppressor transfer RNAs. In a preferred embodiment, the kit comprises a first pharmaceutical composition comprising five suppressor transfer RNAs, a second pharmaceutical composition comprising six suppressor transfer RNAs, the suppressor transfer RNAs in the second pharmaceutical composition being different from the suppressor transfer RNAs of the first pharmaceutical composition, and a third pharmaceutical composition comprising five suppressor transfer RNAs, the suppressor transfer RNAs in the third pharmaceutical composition being different from the suppressor transfer RNAs of the first and second pharmaceutical composition. An example of a kit of the invention is presented in table 1 below.

In a further aspect, the invention relates to a method of treating a person having a disease associated with a nonsense (PTC) mutation, comprising administering an effective amount of a pharmaceutical composition according to the first or third aspect of the invention, or of a synthetic DNA construct according to the second aspect of the invention to the person. In a preferred embodiment the method is for treating Hurler syndrome (MPS I, ICD code E76.0), beta-thalassemia (ICD-10 code D56.1), neurofibromatosis type 1 (NF1, ICD-10 code Q85.0), Duchenne muscular dystrophy (DMD, ICD-10 code G71.0), Crohn's disease (CD, ICD-10 code K50), cystic fibrosis (CF, ICD-10 code E84), neuronal ceroid lipofuscinosis (NCL, ICD-10 code E75.4) spinal muscular atrophy (ICD-10 code G12.9) or Tay-Sachs disease (TSD, ICD-10 code E75.0).

It should be noted that a reference to a tRNA structure (e.g., T arm, AC arm etc.) within a DNA construct is to be understood as a reference to the DNA sequence(s) encoding the structure in the DNA construct. When transcribed into a mature tRNA, the sequences will be transcribed into RNA sequences forming the structure in the mature tRNA.

The invention will be described in the following by way of examples and the appended figures for illustrative purposes only.
Figure 1. Schematic drawing of a generalized "consensus" tRNA structure and its numbering according to tRNA numbering convention.

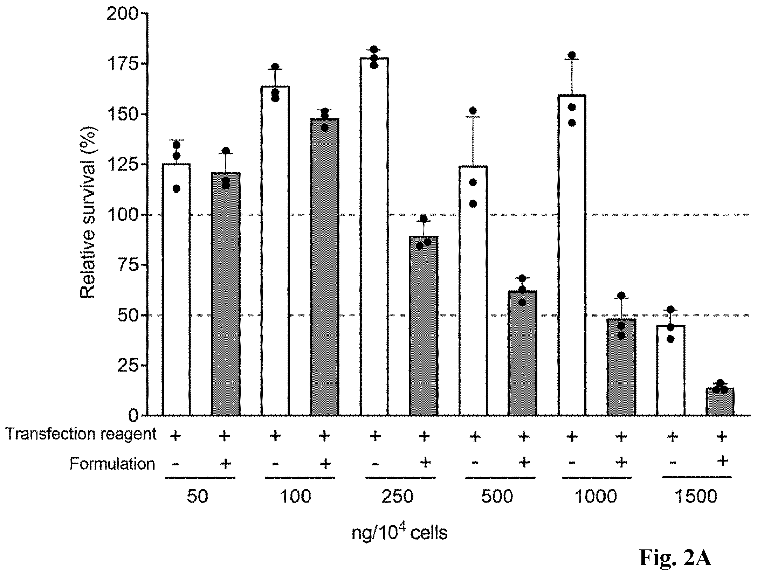

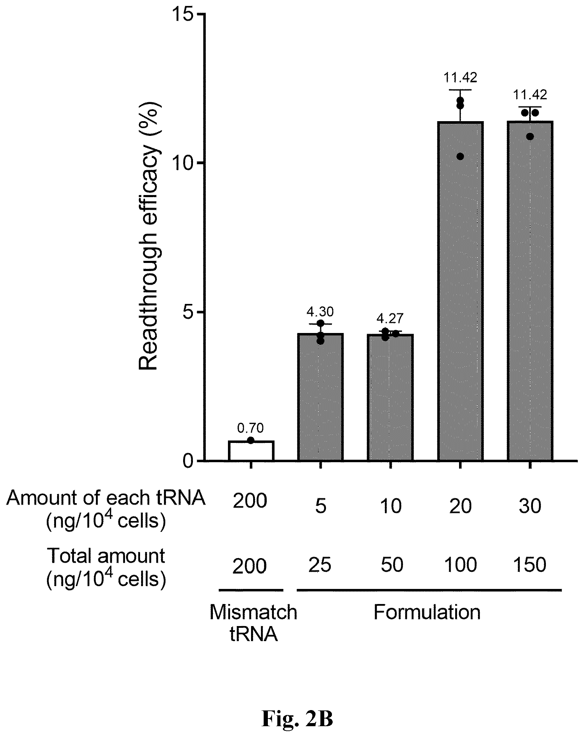

Figure 2. Cell viability and readthrough efficacy of cells treated with different tRNA cocktail concentrations. A. Cell viability assay of cells treated with different tRNA cocktail concentrations. Cells were seeded 1×10⁴ cells/well and to each well different concertation of tRNA was added (ng/10⁴ cells). tRNA-Arg-UGA, tRNA-Ser-UAG; tRNA-Ser-UAA, tRNA-Gln-UAA and tRNA-Cys-UGA were mixed in equimolar concentrations. Cell viability was presented as percentage of the viability of untreated cells which were set as 100%. B. Readthrough efficacy of cells treated with different tRNA cocktail concentrations. Cells were seeded 1×10⁴ cells/well and to each well different concertation of tRNA was added (ng/10⁴ cells). tRNA-Arg-UGA, tRNA-Ser-UAG; tRNA-Ser-UAA, tRNA-Gln-UAA and tRNA-Cys-UGA were mixed in equimolar concentrations. Readthrough activity was presented as percentage of the activity of cells wildtype luciferase (WT-Luc) which were set as 100%.

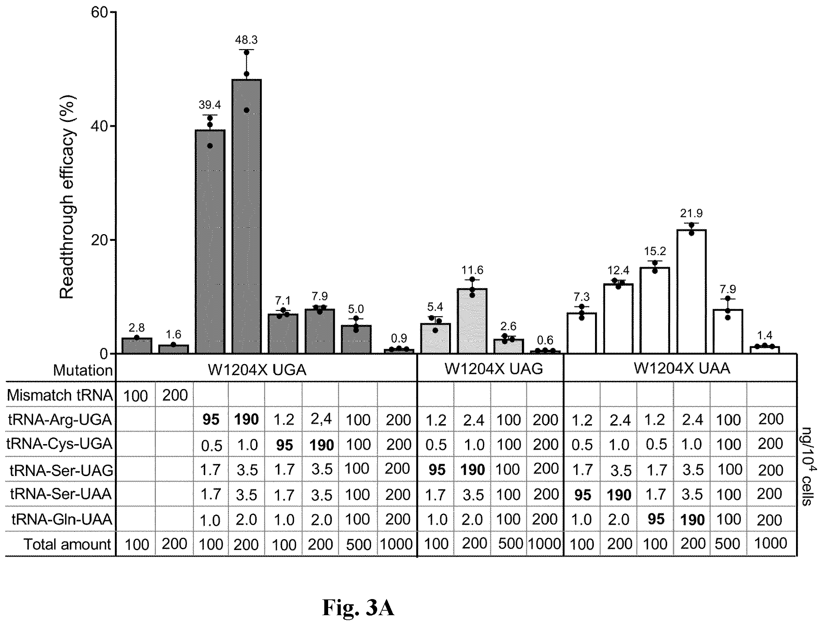

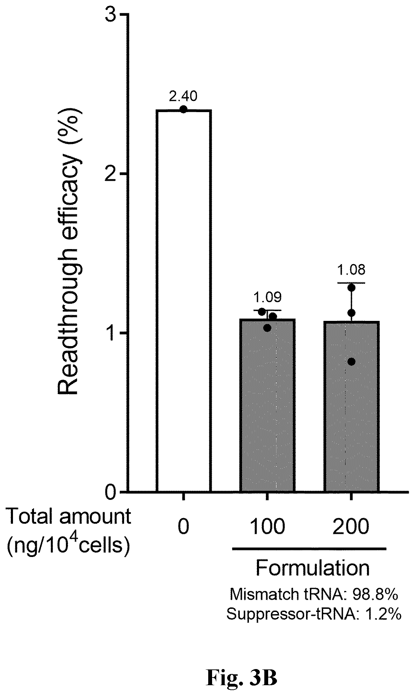

Figure 3. Readthrough efficacy of cells treated with sup-tRNAs. A. Readthrough efficacy of cells treated with different tRNA cocktails with suppressor tRNAs embodiments with different concentrations within each cocktail (numbers under the plot). Cells were seeded 1×10⁴ cells/well and to each well different concertation of tRNA was added (ng/10⁴ cells). Readthrough activity was presented as the percentage of the activity of cells wildtype luciferase (WT-Luc) which were set as 100%. B. Readthrough efficacy of cells treated with tRNA combination containing mismatch tRNA and suppressor tRNA at its lowest concentrations in the embodiment (Figure 3A). Cells were seeded 1×10⁴ cells/well and to each well different concertation of tRNA was added (ng/10⁴ cells). Readthrough activity was presented as percentage of the activity of cells wildtype luciferase (WT-Luc) which were set as 100%. Zero denotes negative control reaction without any tRNA.

Figure 1 depicts an example of a tRNA numbered according to the conventional numbering applied to a generalized "consensus" tRNA, beginning with 1 at the 5' end and ending with 76 at the 3' end. The tRNA is composed of tRNA nucleotides 11 and has the common cloverleaf structure of tRNA comprising an acceptor stem 2 with the CCA tail 10, a T arm 3 with the TψC loop 6, a D arm 4 with the D loop 7 and an anticodon arm 5 with a five-nucleotide stem portion 8 and the anticodon loop 9. In such a "consensus" tRNA the nucleotides of the natural anticodon triplet 25 is always at positions 34, 35 and 36, regardless of the actual number of previous nucleotides. A tRNA may, for example, contain additional nucleotides between positions 1 and 34, e.g. in the D loop, and in the variable loop 24 between positions 45 and 46. Additional nucleotides may be numbered with added alphabetic characters, e.g. 20a, 20b etc. In the variable loop 24 the additional nucleotides are numbered with a preceding "e" and a following numeral depending on the position of the nucleotide in the loop. Positions of modified nucleotides within a tRNA of the invention are marked as black filled circles.

### Examples

In table 1 below an example is presented for customized sup-tRNA compositions ("cocktails") to treat all possible nonsense mutations for CFTR gene; these mutations cause severe cystic fibrosis phenotype. CFTR has 79 PTC mutations (https://cftr2.org/) at codons that encode in total of 9 amino acids, namely: cysteine (UGA), glutamine (UAA/UAG), glycine (UGA), lysine (UAA/UAG), glutamic acid (UAA/UAG), arginine (UGA), serine (UGA/UAG/UAA), tryptophan (UGA/UAG) and tyrosine (UAG/UAA). The identity of the stop codon is given in brackets. In total, 3 cocktails are needed to suppress all mutations. Only these three preparations would have to be approved, but can be used to cure all CF patients with nonsense mutation(s).

**Table 1: Example of a set of three pharmaceutical compositions (sup-tRNA "cocktails") of the invention suitable for the treatment of cystic fibrosis:**

| Cocktail 1 | Cocktail 2 | Cocktail 3 |
|---|---|---|
| tRNAArg (UGA) 5.67% | tRNAGlu (UAG) 6.86% | tRNATrp (UGA) 1.31% |
| tRNASer (UAG) 8.11% | tRNAGlu (UAA) 6.86% | tRNATrp (UAG) 1.31% |
| tRNASer (UAA) 8.11% | tRNASer (UGA) 8.11% | tRNAGln (UAG) 4.65% |
| tRNAGln (UAA) 4.65% | tRNAGly (UGA) 6.60% | tRNALys (UAG) 5.63% |
| tRNACys (UGA) 2.32% | tRNATyr (UAG) 2.75% | tRNATyr (UAA) 2.75% |
| | tRNALys (UAA) 5.63% | |

Each cocktail contains suppressor tRNAs combined the configuration rules of the invention disclosed herein. The percentages given refer to the frequencies of the corresponding amino acids affected by PTCs in the genome (cumulative values are presented for multiple codons encoding the same amino acid). This percentage should be used to determine the ratio of the sup-tRNAs within the cocktails. For example, to introduce arginine at UGA mutation tRNA^{Arg}(TCA) can be used in cocktail 1 at 95% concentration; the rest of the tRNAs within cocktail 1 should be kept at 5% in total following their codon frequency (the percentages given in the table). The choice of the type of suppressor tRNAs in each cocktail is equalized by the number of PTCs of the same type within the CFTR (Cystic Fibrosis Transmembrane Conductance Regulator) gene is implicated in Cystic Fibrosis pathology. In each cocktail, the choice of the suppressor tRNAs was based on their occurrence as PTC mutations in CFTR. For example, in cocktail 1 arginine and serine tRNAs are the highest followed by glutamine, with cysteine being the lowest within the CFTR gene. Similarly, in cocktail 2 Glu has the highest percentage and Lys the lowest, corresponding to PTC frequencies in CFTR. In each cocktail the choice of the suppressor tRNAs was guided by the rationale to adjust the percentages of suppressor tRNAs from high to low according to the PTC mutation frequencies within CFTR from high to low, while avoiding any competition between same codon identities.

### Experimental data

### Material and Methods

tRNAs were transcribed in vitro using T7 transcription system as described in Albers, S., et al. Repurposing tRNAs for nonsense suppression, Nature Comm 12, 3850 (2021). Briefly, two partially overlapping DNA oligonucleotides encoding the corresponding tRNA sequence with an upstream T7 promoter were used for in vitro tRNA synthesis. 24 µM of both oligonucleotides were denatured for 2 min at 95°C, thereafter aligned for 3 min at room temperature in 20 mM Tris-HCl (pH 7.5), 0.4 mM dNTPs were added and incubated with 4 U/µL RevertAid Reverse Transcriptase (Thermo Fisher Scientific) for 40 min at 37°C. This dsDNA template was purified with phenol/chloroform, washed with 80% EtOH and re-suspended in DEPC-H₂O.

HEK293 cells were seeded in 96-well plates at 1×10⁴ cells/well and grown in Dulbecco's Modified Essential Medium (DMEM, Pan Biotech) supplemented with 10% fetal bovine serum (FBS, Pan Biotech) and 2 mM L-glutamine (Thermo Fisher Scientific). 16 to 24 hours later, different concentrations of tRNA embodiments with different concentrations and different proportions of the single tRNA within the cocktails were administered using lipofectamine 3000 (Thermo Fisher Scientific). As a control, the corresponding amount of lipofectamine 3000 (Thermo Fisher Scientific). After four to six hours, medium was replaced and 2X RealTime-Glo^{™} reagent was added to the medium that was replaced following the instructions of cell viability kitRealTime-Glo^{™} MT Cell Viability Assay (Promega) 18 hours post-media change, cells were lysed with 1x passive lysis buffer (Promega) and luciferase activity measured with luciferase assay system (Promega) on the Spark microplate reader (Tecan). Cell viability was presented as the percentage of the viability of untreated cells which were set as 100%.

For activity measurements, the cells were grown the same way. 16 to 24 hours later, cells were co-transfected in triplicate with suppressor tRNA embodiments at different concentrations and combinations along with W1204X PTC-FLuc PTC reporter or WT (wild-type) Fluc plasmids embedded in PGL45.1 backbone and empty control plasmids using lipofectamine 3000 (Thermo Fisher Scientific). The PTC reporter is a luciferase gene without a start codon was extended 5' by 15 codons representing context of a PTC (W1204X, X=UGA). Transfection medium was exchanged after four to six hours. 24 hours post-transfection, the cells were lysed with 1x passive lysis buffer (Promega) and luciferase activity measured with luciferase assay system (Promega) on the Spark microplate reader (Tecan). Readthrough activity was presented as the percentage of the activity of WT luciferase.

### Results

To address the cellular toxicity of tRNA embodiments consisting of multiple suppressor tRNAs, the experiment was performed with a composition, containing five suppressor tRNAs mixed at equal amounts (Figure 2A). At total suppressor tRNA concentration over 500 ng/10⁴ cells less than 50% of the cell viability was retained and at concentration of 1500 ng less than 25%. At concentrations of 50ng/10⁴ cells, 100ng/10⁴ cells, 250ng/10⁴ cells of suppressor tRNAs there was little to no toxicity (Figure 2A), thus it is preferred to maximally use 200-250ng/10⁴ cells. To address the readthrough efficacy of the suppressor tRNAs in the cocktails as in Figure 2A, readthrough at a PTC mutation and rescue of luciferase activity was tested. Comparing to wildtype luciferase, an overall readthrough at maximally 11% was observed. Within this cocktail, each tRNA is included at maximally 30 ng/10⁴ cells, which matches the decoding efficiency of a single operational suppressor tRNA (i.e. the one matching the PTC and amino acid) at that concentration.

To address the readthrough efficacy of the suppressor tRNAs in the cocktails as in Figure 2A, readthrough at a PTC mutation and rescue of luciferase activity was tested. Comparing to wildtype luciferase, an overall readthrough at maximally 11% (Figure 2B) was observed. Within this cocktail, each tRNA is included at maximally 30 ng/10⁴ cells, which matches the decoding efficiency of a single operational suppressor tRNA (i.e., the one matching the PTC and amino acid) at that concentration.

The readthrough efficacy largely increased for the operational suppressor tRNA (i.e., the one matching the PTC and amino acid) at that concentration representing 95% of the total concentration at both cocktails with total tRNA amount of 100 and 200 ng/10⁴ cells (Figure 3A). To test the competitive effect of suppressor tRNAs with anticodon matching the PTC at the low concentrations (Figure 3A), we performed a control experiment, replacing all other tRNAs with non-functional mismatch tRNA. Notably, the functional suppressor tRNA at that concentration is not functional, i.e. with lower readthrough activity than the natural background readthrough (Figure 3B) suggesting that the low concentration of other suppressors matching the PTC do not interfere with the main operational tRNA in the cocktail.

## Claims

1. A pharmaceutical composition comprising at least five different suppressor transfer RNAs, wherein
a) at least one of the suppressor transfer RNAs is able to base-pair to a UGA stop codon, at least one of the suppressor transfer RNAs is able to base-pair to a UAA stop codon, and at least one of the suppressor transfer RNAs is able to base-pair to a UAG stop codon; and
b) the suppressor transfer RNAs are not all present in the composition in the same amount; and a pharmaceutically acceptable carrier.

2. The pharmaceutical composition according to claim 1, wherein the at least five suppressor transfer RNAs are selected from the group of suppressor transfer RNAs consisting of: tRNA-Arg-UR¹R², tRNA-Ser-UR¹R², tRNA-Gln-UR¹R², tRNA-Cys-UR¹R², tRNA-Glu-UR¹R², tRNA-Gly-UR¹R², tRNA-Tyr-UR¹R², tRNA-Lys-UR¹R², tRNA-Trp-UR¹R², and tRNA-Leu-UR¹R², wherein R¹ and R² are, independently from each other, A or G, with the proviso that R¹ and R² are not both G, preferably selected from the group of suppressor transfer RNAs consisting of: tRNA-Arg-UGA, tRNA-Ser-UAG, tRNA-Ser-UAA, tRNA-Ser-UGA, tRNA-Gln-UAA, tRNA-Gln-UAG, tRNA-Cys-UGA, tRNA-Glu-UAG, tRNA-Glu-UAA, tRNA-Gly-UGA, tRNA-Tyr-UAG, tRNA-Tyr-UAA, tRNA-Lys-UAA, tRNA-Lys-UAG, tRNA-Trp-UGA, tRNA-Trp-UAG, tRNA-Leu-UGA, tRNA-Leu-UAA, and tRNA-Leu-UAG.

3. The pharmaceutical composition according to claim 1 or 2, wherein the composition comprises no more than three suppressor transfer RNAs base-pairing to the same stop codon, with the proviso that, if the composition contains more than one suppressor transfer RNA base-pairing to the same stop codon, the suppressor transfer RNAs base-pairing to the same stop codon differ in their cognate amino acid, and, preferably have tRNA bodies, or parts thereof, from non-cognate tRNAs only.

4. The pharmaceutical composition according to one of the preceding claims, wherein the composition comprises five or six suppressor transfer RNAs, preferably no more than five or six suppressor transfer RNAs, and wherein the composition comprises the following suppressor tRNAs:
a) tRNA-Arg-UGA, tRNA-Ser-UAG, tRNA-Ser-UAA, tRNA-Gln-UAA, and tRNA-Cys-UGA; or
b) tRNA-Glu-UAG, tRNA-Glu-UAA, tRNA-Ser-UGA, tRNA-Gly-UGA, tRNA-Tyr-UAG, and tRNA-Lys-UAA; or
c) tRNA-Trp-UGA, tRNA-Trp-UAG, tRNA-Gln-UAG, tRNA-Lys-UAG, and tRNA-Tyr-UAA.

5. The pharmaceutical composition according to one of the preceding claims, wherein the proportion of each suppressor transfer RNA within the composition is adapted to the frequency of PTC mutations in a given gene of interest, such that a first suppressor transfer RNA configured to suppress a PTC with the highest frequency is present in the composition with the largest proportion, and a second, third, fourth and further suppressor transfer RNA configured to suppress a PTC with a lower frequency is present in the composition with a corresponding lower proportion, depending on the frequency of the targeted PTC in the gene of interest.

6. The pharmaceutical composition according to claim 5, wherein the proportion of the suppressor transfer RNAs present in the composition targeting a PTC in the gene of interest having a frequency of at least 0,5 %, is at least 95% by weight of the entirety of the suppressor transfer RNAs present in the composition.

7. A synthetic DNA construct comprising a) at least five nucleic acids each encoding a different suppressor transfer RNA and b) DNA regulatory elements functionally linked to the nucleic acids in such a manner, that at least one of the five nucleic acids can be expressed independent of the other nucleic acids in the construct.

8. A pharmaceutical composition comprising a) at least five different synthetic DNA constructs, wherein each of the synthetic DNA constructs comprises a nucleic acid encoding a different suppressor transfer RNA and a DNA regulatory element functionally linked to the nucleic acid in such a manner, that at least one of the five nucleic acids in the pharmaceutical composition can be expressed at an expression level different from the expression level of the other nucleic acids in the composition, or wherein at least one of the at least five different synthetic DNA constructs is present in the pharmaceutical composition in a higher copy number than the other synthetic DNA constructs, and b) a pharmaceutically acceptable carrier.

9. The synthetic DNA construct according to claim 7 or the pharmaceutical composition according to claim 8, wherein the at least five nucleic acids each encode a different transfer RNA, and wherein at least one, two, three, four, five or all of the nucleic acids are each functionally linked to a 5' leader sequence, the 5' leader sequence comprising
a) at least one sequence motif selected from the group consisting of the sequence motifs TGACCTAAGTGTAAAGT, I_{H}, (SEQ ID NO: 28), TGAGATTTCCTTCAGGTT, II_{H}, (SEQ ID NO: 29), TATATAGTTCTGTATGAGACCACTCTTTCCC, III_{H}, (SEQ ID NO: 30), ACCATAAACGTGAAATG, I_{L}, (SEQ ID NO: 31), TCTTTGGATTTGGGAATC, II_{L}, (SEQ ID NO: 32, and TTATAAGTTCTGTATGAGACCACTCTTTCCC, III_{L}, (SEQ ID NO: 33); or
b) one sequence motif selected from the group consisting of the sequence motifs VNANANTVHANANNTTNNNATRANATTCNGDGVGNAANATABTNCTVGND, 50nt_{H}, (SEQ ID NO: 34) and GCNGGDGGCGNGTTCBBCTGTNVNAGCNTNCGDNGNCGTTTNNCNTCGGT, 50nt_{L}, (SEQ ID NO: 35); or
c) at least one sequence motif selected from the group consisting of the sequence motifs GAAATGCCTT, 10nt_{H1}, (SEQ ID NO: 36), GTGGGAACTA 10nt_{H2}, (SEQ ID NO: 37), and GTGTTGCTTG 10nt_{H3}, (SEQ ID NO: 38);
and wherein at least one of the nucleic acids encoding a suppressor transfer RNA has a 5' leader sequence different from the other nucleic acids encoding a suppressor transfer RNA.

10. The synthetic DNA construct or the pharmaceutical composition according to claim 9, wherein
aa) in relation to a) above,
i. the 5' leader sequence does not comprise the sequence motif I_{H} if it comprises the sequence motif I_{L}, and vice versa, and wherein, if the 5' leader sequence comprises one of the sequence motifs I_{H} or I_{L}, the sequence motif I_{H} or I_{L} has, in 5'-3' direction, preferably the positions -66 to -50;
ii. the 5' leader sequence does not comprise the sequence motif II_{H} if it comprises the sequence motif II_{L}, and vice versa, and wherein, if the 5' leader sequence comprises one of the sequence motifs II_{H} or II_{L}, the sequence motif II_{H} or II_{L} has, in 5'-3' direction, preferably the positions - 49 to -32, and
iii. the 5' leader sequence does not comprise the sequence motif III_{H} if it comprises the sequence motif III_{L}, and vice versa, and wherein, if the 5' leader sequence comprises one of the sequence motifs III_{H} or III_{L}, the sequence motif III_{H} or III_{L} has, in 5'-3' direction, preferably the positions -31 to -1; or
bb) in relation to b) above, the 5' leader sequence does not comprise the sequence motif 50nt_{H} if it comprises the sequence motif 50nt_{L}, and vice versa, and wherein, if the 5' leader sequence comprises one of the sequence motifs 50nt_{H}, or 50nt_{L}, the sequence motif 50nt_{H} or 50nt_{L} has, in 5'-3' direction, preferably the positions -50 to -1.

11. The synthetic DNA construct or the pharmaceutical composition according to one of claims 9 to 10, wherein the 5' leader sequence comprises,
aa) in relation to a) above, at least two sequence motifs selected from the sequence motifs I_{L}, II_{L}, III_{L}, I_{H}, II_{H} or III_{H}, and wherein the at least two sequence motifs are arranged, in 5'-3' direction, in the order I_{H} - II_{H}, II_{H} - III_{H}, I_{H} - III_{H}, I_{L} - II_{L}, II_{L} - III_{L}, I_{L} - III_{L}, I_{H} - II_{L}, I_{H} - III_{L}, II_{H} - III_{L}, I_{L} - II_{H}, II_{L} - III_{H}, or I_{L} - III_{H}; or
cc) in relation to c) above, at least two sequence motifs of the sequences motifs 10nt_{H1}, 10nt_{H2}, and 10nt_{H3}, the at least two sequence motifs being:
i. GAAATGCCTT, 10nt_{H1}, (SEQ ID NO: 9) and GTGTTGCTTG, 10nt_{H3}, (SEQ ID NO:11), wherein the sequence motifs are arranged, in 5'-3' direction, in the following order: 10nt_{H3} - 10nt_{H1}, or
ii. GAAATGCCTT, 10nt_{H1}, (SEQ ID NO: 9) and GTGGGAACTA, 10nt_{H2}, (SEQ ID NO:10), wherein the sequence motifs are arranged, in 5'-3' direction, in the following order: 10nt_{H1} - 10nt_{H2}, or
iii. GTGGGAACTA, 10nt_{H2}, (SEQ ID NO:10), and GTGTTGCTTG, 10nt_{H3}, (SEQ ID NO:11), wherein the sequence motifs are arranged, in 5'-3' direction, in the following order: 10nt_{H3} - 10nt_{H2}.

12. The synthetic DNA construct or the pharmaceutical composition according to one of claims 9 to 11, wherein the 5' leader sequence comprises
aa) in relation to a) above, three sequence motifs selected from the sequence motifs I_{L}, II_{L}, III_{L}, I_{H}, II_{H} or III_{H}, preferably in the following order, in 5'-3' direction: I_{L} - II_{L} - III_{L}, I_{H} - II_{H} - III_{H}, I_{H} - II_{L} - III_{L}, I_{L} - II_{H} - III_{L}, I_{L} - II_{L} - III_{H}, I_{H} - II_{H} - III_{L}, I_{H} - II_{L} - III_{H}, or I_{L} - II_{H} - III_{H}; or
cc) in relation to c) above all three sequence motifs 10nt_{H1}, 10nt_{H2}, and 10nt_{H3}, preferably in the following order, in 5'-3' direction: 10nt_{H3} - 10nt_{H1} - 10nt_{H2}.

13. The synthetic DNA construct or the pharmaceutical composition according to one of claims 9 to 12, wherein the 5' leader sequence has or comprises
aa) in relation to a) above, a sequence according to one of SEQ ID NO: 39-64
bb) in relation to b) above, a sequence according to one of SEQ ID NO: 65-78.

14. The pharmaceutical composition according to one of claims 1 to 6, the synthetic DNA construct according to one of claims 7, 9-13, or the pharmaceutical composition according to one of claims 8 to 13 for use as a medicament.

15. The pharmaceutical composition according to one of claims 1 to 6, the synthetic DNA construct according to one of claims 7, 9-13, or the pharmaceutical composition according to one of claims 8 to 13 for use as a medicament in a disease, which is at least partly caused by a premature termination codon, PTC, leading to the production of a protein being dysfunctional or non-functional compared to the wild-type protein.
